# EUROPEAN PATENT SPECIFICATION

(11) **EP 2 564 898 B1**
(45) Date of publication and mention of the grant of the patent: **29.04.2015**
(21) Application number: 11179726.2
(22) Date of filing: 01.09.2011
(51) Int. Cl.: A61B 5/00, A61N 5/06

(54) **Device for a controlled and safe irradiation to the human body and apparatus making use of the device**
Vorrichtung für eine gesteuerte und sichere Bestrahlung des menschlichen Körpers und die Vorrichtung verwendendes Gerät
Dispositif pour irradiation contrôlée et sécurisée du corps humain et appareil utilisant le dispositif

(43) Date of publication of application: 06.03.2013
(73) Proprietor: JK-Holding GmbH, 53578 Windhagen (DE)
(72) Inventor: Gerstenmeier, Jürgen, 53578 Windhagen (DE); Nanninga, Herman, 53578 Windhagen (DE)
(74) Representative: Koepe, Gerd L.

(56) References cited:
- EP-A2- 1 508 301
- WO-A2-2007/019536
- US-A1- 2008 139 901
- US-A1- 2008 319 430

## Description

The present invention relates to a device for a controlled and safe irradiation, to the human body, with actinic radiation. In particular, the invention relates to a device for irradiating the human body with actinic radiation in a physiologically effective wavelength region, more particularly with actinic radiation containing a certain portion of ultraviolet (UV) radiation, e. g. tanning radiation, and/or with actinic radiation containing a certain portion of visible and/or near-infrared radiation. The device takes into account that different persons have different types of skin and experience different sensitivities of their skin against physiologically effective radiation (e. g., without restriction, tanning radiation and/or keratinogenic radiation and/or warming radiation) and may be used to being exposed to such actinic radiation, in particular to radiation having a certain portion of UV radiation and/or of visible radiation and/or of IR radiation, to different degrees. The invention also relates to an apparatus making use of the device of the present invention.

Tanning apparatus based on UV radiators are known in many embodiments and sizes. Particularly popular are tanning apparatus allowing tanning of the whole human body. Such tanning apparatus comprise a lower portion comprising a bed ("bench") optionally provided with a profile and having installed a number of UV radiator tubes below a surface transparent for tanning radiation, which tubes emit UV radiation at least a part of which has a tanning effect. The transparent surface, beyond protecting the body of a user from directly contacting the actinic radiation-emitting tubes, is used by the user of the tanning apparatus to place on it at least a part of the body, usually the back side of the whole body, so as to be in a position for receiving said actinic radiation. The lower portion of the tanning apparatus corresponds to a movable upper portion either pivotable around an axis parallel to one of the edges of the bed, usually parallel to one of its long edges, or movable in a vertical direction on a stand or on a suspension fixed to the ceiling of a room. The upper portion has also installed a number of UV radiator tubes - above a lower surface transparent for tanning radiation - which tubes emit UV radiation at least a part of which has a tanning effect. Users of the tanning apparatus lift or swivel the upper portion thereof into an open position allowing a convenient getting onto the bed, lay down on the bed, close the upper portion and then switch on the UV radiator tubes for a preselected tanning time. Such time may be selected in a time range of a few minutes, but may also be a time period of 15 minutes or even more. A tanning process by making use of the above-mentioned apparatus described only with respect to important general features is also known and, hence, is state of the art.

As known to a skilled person, apparatus for irradiating actinic radiation (e. g. UV radiation at least a part of which has a tanning effect) are also known wherein (or whereon) a user may receive an irradiation dose for a tanning effect in a sitting or standing position on a part/on parts or on the whole of the body.

In the case of tanning, such devices as described above are used to an increasing extent both in the cosmetic field (as a private tanning application at home or in so-called "tanning studios" providing professionally operated tanning devices for tanning the skin of a user, i.e. a human) and in the medical field, where a preventive or therapeutic treatment of the skin is more and more used successfully as a drug-free treatment route or as a treatment route accompanying a drug-involving treatment of the skin (for example in cases of acne, psoriasis etc.).

Basically, prior art tanning apparatus are designed in such a manner that a tanning process may be performed without the human skin being damaged by components of the radiation emitted by the radiator tubes, in particular not by UV radiation components, provided that habits and protection measures are maintained which a person undergoing a tanning process has to observe also in cases when exposing himself/herself to natural sun radiation. In other words: In the same way as when tanning in nature, a person having a "white" skin and/or having had few previous opportunities of being exposed to natural sun radiation and/or artificial UV radiation (e. g. UV radiation of a tanning apparatus) has to select lower radiation doses and/or shorter irradiation times and/or has to protect the skin more carefully with usual (e. g. cosmetic) sun-screens than a person having a "darker" skin and/or having had more previous opportunities of being exposed to the radiation of the sun and/or to artificial UV radiation.

In the cosmetic field, in particular when tanning apparatus are provided on a professional basis, usually advisors are available to advise the customer or user with respect to the tanning time and/or irradiation dose appropriate in view of the customer's/user's skin status. Often, however, such advice is not accepted. In the private area, such advice is not available at all. Quite to the contrary: The person intending to use the tanning apparatus selects, on his/her own volition, the tanning time and irradiation dose of the apparatus in most of the cases. Hence, even in cases where skilled advice was given before, this does not reliably prevent a stronger irradiation dose or a longer tanning time, respectively, from being selected and applied, for example in erroneously desiring to accelerate the process of tanning or to achieve a darker tan within one treatment "session". As is known from nature, excessive exposure to UV actinic radiation may result into erythemas caused on the skin by the UV radiation, which erythemas are known under the term "sunburn" and which may be very painful and may severely damage the skin in the long run.

The document DE-A 43 12 547 describes tanning devices allowing the risks of a too intensive irradiation of the skin to be diminished substantially both in the medical field and in the cosmetic field. This may be accomplished, according to DE-A 43 12 547, by means of an adaptation of the tanning UV radiation of a tanning device to the individual type of skin of the respective user. The UV radiator tubes are provided with separate means for controlling their power, and several of those power control means are connected to a central control means allowing an individual control of single radiator tubes. Practically, the power control is achieved by a means for determining the individual skin color of the person to be subjected to irradiation (which means has per se been known for a long time). Immediately on the skin of said person, the type of skin and the skin color, respectively, of the person to be irradiated are detected optically. The detection is performed by irradiating light of a well-defined spectral composition, scanning the light reflected by the skin by means of a scanning head and determining the ratio of the light absorbed/reflected to the light irradiated. The data obtained (and compared to empirical data) are fed to the central processing unit, and the maximum irradiation dose which the person to be irradiated may receive is determined, as is also determined - depending upon the desired duration of the irradiation - the maximum irradiation time applicable in each individual case.

A disadvantage of the above proposal is that it is not taken into account that different parts of the human body are susceptible to tanning radiation to a different degree. Moreover, different parts of the human body have a different sensitivity to tanning UV radiation. For example, the body (and in particular, those parts of the body which usually are covered by clothes) are considerably more sensitive to UV radiation than the face. A measurement of the skin tan or the skin type, respectively, at the face of a user by means of the device described in the document DE-A 43 12 547 regularly results into a higher maximum dose of allowed radiation than a measurement of the skin tan or skin type, respectively, at the body, for example at the chest or at the belly. The consequences of such "mis-measurement", although correct for the skin type measured actually, are too low (what would not be harmful) or too high radiation doses at other parts of the skin of the same user. Specifically for the above-mentioned case of a primary measurement of the skin type or the skin tan, respectively, at one of the (usually) less sensitive parts of the skin (e.g. at the face), the body is (and more sensitive parts of the body are) subjected to a too strong dose of tanning radiation.

Such an above erythema-threshold irradiation of the skin results into skin irritation or even into erythemas and, in the worst case, into a severe damage of sensitive skin in a long-run view.

In order to avoid such "mis-measurements" inevitably occurring in accordance with the above prior art, and particularly in order to prevent users from intentionally circumventing measures for restricting the irradiated actinic radiation dose, a process for irradiating radiation comprising UV radiation to a user's body was proposed in the document EP-A 1 508 301, said process comprising the step of measuring the status, as far as relevant for determining the UV irradiation dose, of the user's skin at several points of the skin/cutis, and controlling the power of single groups of said UV radiation sources, which can direct their radiation substantially separately onto different parts of the user's body, in accordance with the results of said measurements.

In accordance with the proposal of the document EP-A 1 508 301 (and in contrast to the above prior art), it was possible to directly control the UV radiation emission per surface (including user's skin surface) area (i. e. the irradiance of the actinic radiation) by the results of the skin status and skin color measurements. Given a certain fixed time of irradiation of the actinic radiation (i. e. of irradiation of the UV radiation) to the user's body, the total amount of UV radiation received by the user could be controlled not to exceed the erythema threshold level appropriate to a user having a certain skin tan status or skin color and could not be influenced intentionally by the user. By such an apparatus and method for controlling the actinic radiation applied, it was successfully possible to prevent the user from suffering any damage. Even more, manufacturers of tanning apparatus had at hands an effective measure for guarantying that, if applied as proposed, the legally proposed erythema threshold irradiance value of E_{R} = 0.3 W/m² is not exceeded for a certain user at any time.

When irradiating the human body with actinic radiation not in the UV wavelength region, no maximum irradiance values must be observed, by now, by manufacturers and professional and private operators of the respective apparatus, as they are obligatory for tanning devices (i. e. devices irradiating actinic radiation in the UV wavelength range). It is, however, assumed to be applicable that also in wavelength ranges other than UV wavelengths, a certain irradiance value should not be exceeded in all cases where the radiation has a physiological effect on the user. Hence, a control of actinic radiation irradiation to the human body was proposed, and is observed, also in ranges of wavelengths longer than the UV part of the actinic radiation spectrum, e. g. in the visible wavelengths range of, for example, 450 to 800 nm, and/or in the IR wavelengths range of, for example, > 800 nm, of which the wavelengths ranges of from 570 nm to 610 nm, from 610 nm to 650 nm, from 650 nm to 690 nm and/or from 740 nm to 780 nm and/or from 810 nm to 850 nm, are typical, but not restricting examples.

In the above-referenced prior art document EP-A 1 508 301, the status and/or color, as far as relevant for determining the UV irradiation dose, of a person's skin is measured ahead of the start of the UV irradiation. This is effected by means of a device which comprises, and in a preferred embodiment consists of, a means emitting radiation having a defined spectral composition onto the skin (cutis) of the user desiring to be subjected to UV irradiation; a means of measuring the portion of the radiation irradiated by the emission means and reflected (or absorbed) by the user's skin (cutis); and means for transmitting measured data of emitted radiation to reflected (or absorbed) radiation (or calculated data of the ratio of emitted radiation to reflected (or absorbed) radiation) to the means for controlling the irradiance of UV radiation-emitting lamps directly and without any possibility of influence by the person subjected to UV radiation.

In order to achieve a desired precision of the emission and reflection/absorption measurement, on the one hand, and to facilitate the practical operation of the measuring device while being contacted to the user's skin (cutis), all parts of said prior device were accommodated in a measuring head or sensor allowing a fixed arrangement of light emitting LEDs, the Ulbricht sphere (used as the diffusor for the light emitted by the LEDs) and the sensor receiving the light reflected from (and not absorbed by) the user's skin (cutis). However, in order to avoid extraneous light to distort the measurement of the skin status or skin color, the edges of the surface of the measuring head contacting the user's skin were made tight by a sealing rim or lip made of a flexible material, e. g. rubber or another (synthetic) polymer. The latter was a "weak part" of the measuring head: The sealing rim (or lip) surrounding the surface of the measuring head contacting the user's skin had to be disinfected, and relieved from the user's body fat and sweat, after every use. Moreover, the sealing rim (or lip) was subjected to pressing forces in the course of each skin status measurement several times. Hence, it had to be replaced often due to wear and erosion, and gave rise to mis-measurements in the meantime.

Moreover, the components of the measuring head were neither in a fixed relation to each other nor in relation to the surface of the user's skin, which fact was found to be detrimental due to the fact that the precision of the measurement depends upon the length of the irradiated and reflected light's pass. Although the changes of the relative distances were only in the order of a fraction of a millimeter, this gave rise to erroneous results, too.

Hence, the desired results of a sufficiently accurate measurement of the user's skin status or skin color could not be guaranteed for each measurement. Thus, an improvement of the means for measuring the user's skin status and/or skin color was eagerly desired, also in view of the fact that such a measurement is expected to be needed in the future in cases of irradiating the human body with actinic radiation of the non-UV ranges, too.

The invention relates to a device for a measurement of the status and/or color, as far as relevant for determining an applicable actinic irradiation dose, of the skin of a human on his/her skin (cutis), comprising, housed in a rigid housing,
(a) at least one means for emitting radiation having a defined spectral composition onto the skin (cutis);
(b) at least one sensor for measuring the portion of the radiation irradiated by the radiation-emission means which is reflected by the skin (cutis); and
(c) at least means for providing energy to the at least one means and/or to the at least one sensor and for transmitting measured data to a data analyzing means,
   said device further comprising
(d) in said housing: at least one means for diffusing the radiation, emitted by the radiation emission means, between the radiation emission means and the human's skin (cutis);
(e) in said housing: at least one means for focusing, towards the at least one sensor, the portion of the radiation irradiated by the radiation-emission means which is reflected by the skin (cutis);
(f) in said housing: at least one means capable of fixing the position of said at least one radiation emission means, said at least one focusing means, said at least one sensor and said at least one radiation diffusion means relative to each other; and
(g) at least one flat and rigid surface area capable of allowing the passage of the radiation emitted from said at least one radiation emission means out of said housing towards the human's skin (cutis) and of the radiation reflected from the human's skin (cutis) into said housing towards the at least one focusing means and the at least one sensor,
   said at least one flat and rigid surface area being included into the arrangement of the four components of (f) above fixed by said fixing means; said device further comprising (h) a flexible membrane which incorporates said flat and rigid surface area, and (i) at least one means (209) disposed within the housing (200 a) and capable of actuating the human's skin status and/or skin color measurement.

Preferred embodiments of the device of the invention are claimed in the claims dependent upon claim 1.

The invention further relates to an apparatus for irradiating actinic radiation onto the body of a user, said apparatus comprising at least one means having a surface facing the user and being transparent for the actinic radiation and having several groups of actinic radiation sources arranged on that side of the surface which is turned away from the user, which actinic radiation sources are designed, with respect to their number and position, in such a way that they can direct actinic radiation substantially separately onto different parts of the user's body, and are controllable by one or more means for controlling the power of the actininc radiation sources, wherein the power control is effected in accordance with the results of the measurements of the status, as far as relevant for determining an applicable actinic irradiation dose, of the user's skin at several points of his/her cutis, by means of at least one device according to the present invention as described in detail below.

Preferred embodiments of such an apparatus are claimed in the claims dependent upon claim 7.

The invention also relates to a device as described in detail below for use in a process for irradiating, to a user's body, actinic radiation from actinic radiation sources emitting such radiation, said device being capable of enabling the steps of measuring the status and/or the color, as far as relevant for determining an applicable actinic irradiation dose, of the user's skin at several points of the skin (cutis), and of controlling the power of single groups of said actinic radiation sources, capable of directing their radiation substantially separately onto different parts of the user's body, in accordance with the results of said measurements effected by means of said device described below in detail.

Preferred embodiments of the device for use in said process are claimed in the claims dependent upon claim 14.

The present invention is now further explained in detail by means of its preferred embodiments, which are given for exemplary purposes in order to allow a better understanding of the invention, but should not be construed to restrict the invention to only these preferred embodiments.

The terms "subject", "person", "human" and "user" are used in the present specification and claims synonymously and mean the person who uses the device of the present invention for controlling a process of being irradiated by the actinic radiation; of course, the person is subject to control of such irradiation by the device of the invention.

The term "actinic light" or "actinic radiation" as used in the present specification and claims is understood to mean light or (broader) radiation of the whole electromagnetic spectrum (see the definition with reference to "Rompp Chemie-Lexikon") which has a photochemical (including a photobiochemical) effect and includes, as the case may be, light/radiation having natural or artificial origin. In the claims and specification, "actinic light" or "actinic radiation" is mainly, but not restrictively, used for light or radiation having an artificial origin.

The present invention is further described by referring to Figures. These Figures show preferred embodiments of the invention and are given for allowing a better understanding of the invention. However, the invention is not restricted by these preferred embodiments. In the Figures,
Figure 1 shows one embodiment of a device for measuring the status, as far as relevant for determining the actinic irradiation dose, of a person's skin, particularly for measuring the skin color and/or the skin status of a person in a perspective view;
Figure 2 shows a sectional perspective view of the device for measuring the status and/or the color, as far as relevant for determining the actinic irradiation dose, of a person's skin, with at least a part of the relevant mechanical and electronic details opened to the viewer, wherein the reference numerals have the following meaning:
   - 200a: housing;
   - 200b: electronics;
   - 230: surface elements showing the status of the measurement;
   - 210: magnets for switching;
   - 220: electro-optical measuring sensor and lens;
   - 208: membrane;
   - 202: measuring sensor;
   - 212: connecting line;
   - 209: photodiode, Hall sensor;

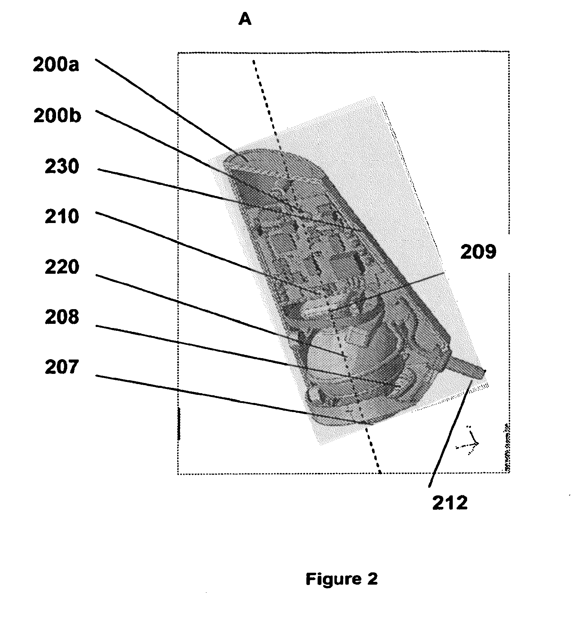

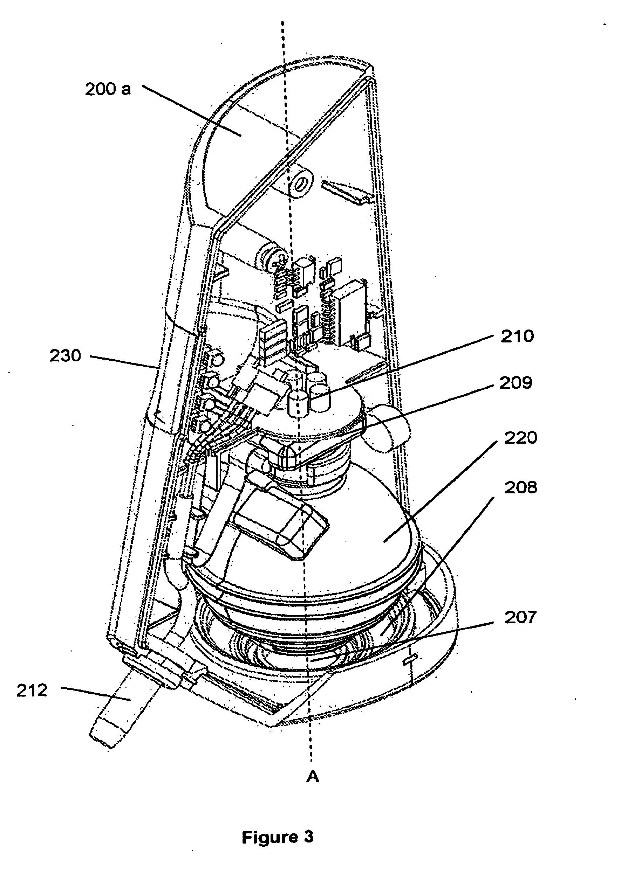

Figure 3, similar to Figure 2, shows a sectional perspective view of the device for measuring the status and/or the color, as far as relevant for determining the actinic irradiation dose, of a person's skin, with at least a part of the relevant mechanical and electronic details opened to the viewer;

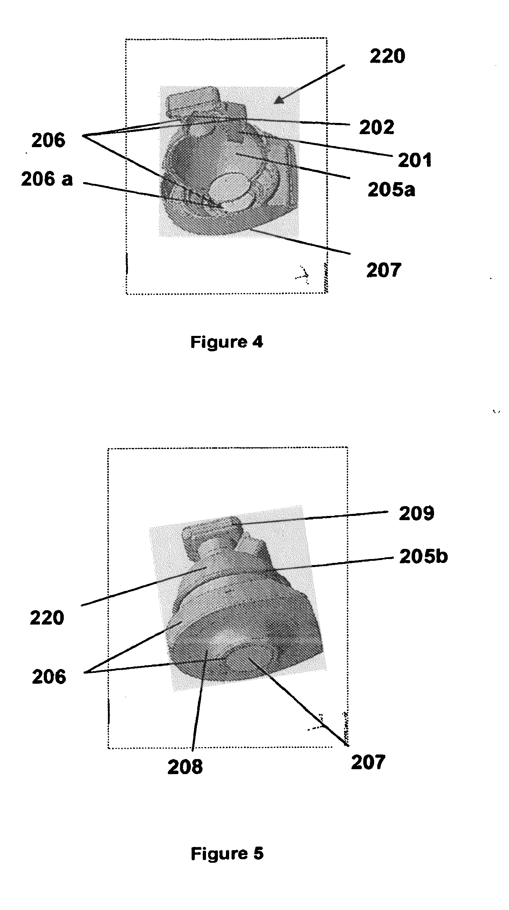

Figure 4 shows a perspective (partially opened) view to the electro-optical measuring sensor 220 in a perfect parallel arrangement to the measuring surface area 207 due to the relative fixation by the fixation means 206;
Figure 5 shows a perspective view to the electro-optical measuring sensor 220 in relative (fixed) arrangement to the membrane 208 and to the measuring surface area 207;

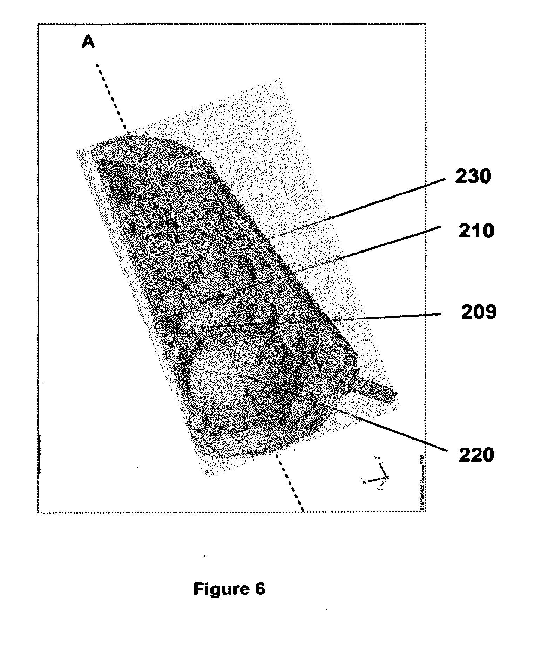

Figure 6 shows a full perspective view, similarly to Figure 2, to the device 200 comprising particularly the Hall sensor 209 and the magnets 210 for switching;

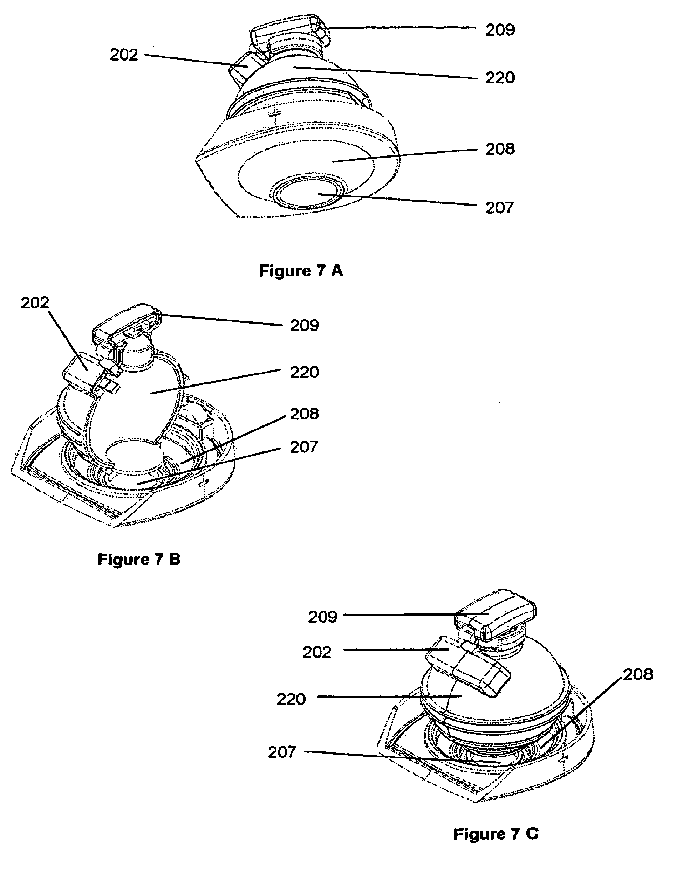

Figures 7A, 7 B and 7 C show perspective views (in one case: Figure 7 B: partially opened) to the electro-optical measuring sensor 220 and the additional adjacent components of the device 200 of the invention; and

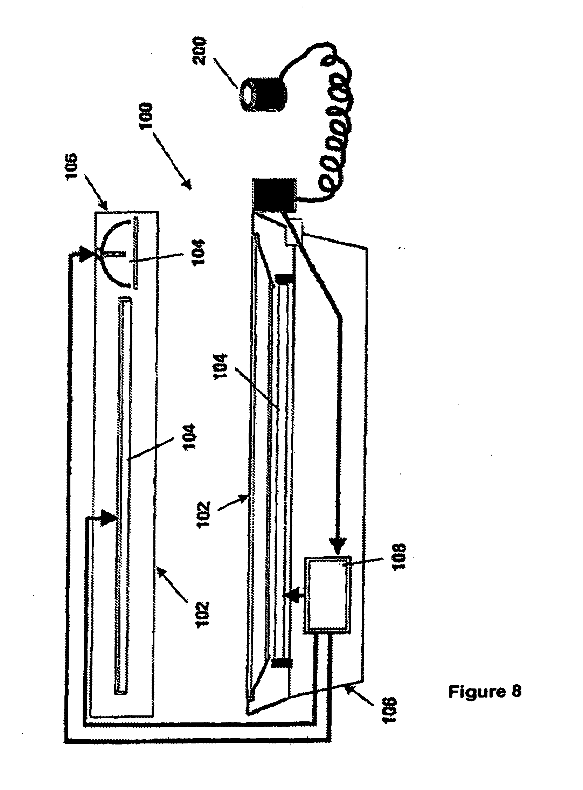

Figure 8 shows a scheme of a specific embodiment of an apparatus for irradiating actinic radiation onto the body of a user.

The invention is now explained in detail by making reference to its preferred embodiments shown in the Figures.

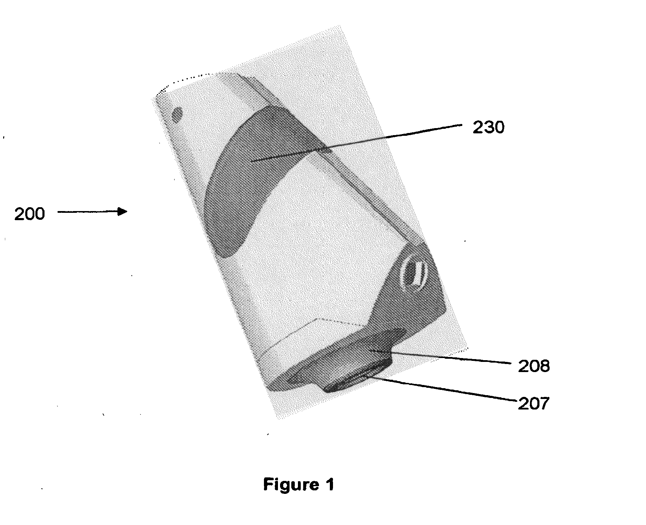

Reference is now made to Figure 1. Figure 1 shows one embodiment of a device 200 for measuring the status, as far as relevant for determining the actinic irradiation dose, of a person's skin (cutis), particularly for measuring the skin color and/or the skin status of a person, in a perspective view.

The term "skin color" is understood, in the context of the present invention, as the sum of all wavelengths of reflected light which is reflected from the skin surface or cutis of a person, who may also be referred herein as "user" (i.e. a user of the device or of the apparatus of the present invention, for example, which terms "person" and "user" are used in the following synonymously), as a consequence of an irradiation of light having a defined intensity and wavelength. The skin color serves as a basis for evaluating the question as to which actinic radiation dose, for example (but without restriction) which ultraviolet radiation dose and/or visible radiation does and/or infrared radiation dose, can be tolerated by the skin without it being irritated by an erythema or harmed by other undesired skin-damaging conditions, not even in its first signs. It was found that the skin of different persons reacts in different ways to light having a defined spectral composition and being irradiated onto the skin for a measurement of the skin status, as far as relevant for determining the actinic irradiation dose. The skin of persons having a white skin color usually reacts in a clearly more sensitive way (and, hence, may tolerate clearly less actinic radiation before reaching the erythema (or other undesired skin condition) irritation threshold) than the skin of people having a dark skin color. The light reflected by the surface (cutis) of the skin having a specific skin color provides a good measure for the skin sensitivity towards actinic radiation as it is used for the process of irradiating the skin with actinic radiation.

In contrast to the term "skin color", the term "skin status" is understood to mean in the context of the present invention, the status of the skin at the time of measurement, as caused by a previous actinic radiation irradiation treatments. One specific example of a consequence of such an actinic radiation irradiation treatment is skin pigmentation which is achieved as a reaction to the irradiation of the skin by UV radiation. It is known from previous experience that the pigmented (dark) skin is less sensitive to actinic radiation as, for example (but without restriction) to UV radiation, than less pigmented (white) or even unpigmented skin. Independent from the skin color, the skin status, for example the skin tan status or, more generally, the skin pigmentation status, provides another good measure for the actinic radiation dose, which the skin of a person may receive without being irritated by erythemas.

The device 200 according to the invention for a measurement of the status, as far as relevant for determining the actinic irradiation dose, of the skin of a person and - particularly preferred - for a measurement of the skin color and/or of the skin status of a person, is shown in figures 1 to 4. In general, the skin color of a person and/or the skin status of a person is/are measured by means of a device 200 comprising, housed in a rigid housing 200 a, (a) at least one means 201 for emitting radiation having a defined spectral composition onto the skin (cutis); (b) at least one sensor 202 for measuring the portion of the radiation irradiated by the radiation-emission means 201 which is reflected by the skin (cutis); and (c) at least means 212 for providing energy to the means 201 and/or to the sensor 202 and for transmitting measured data to a data analyzing means 203; said device further comprising (d) in said housing 200 a: at least one means 205 for diffusing the radiation, emitted by the radiation emission means 201, between the radiation emission means 201 and the human's skin (cutis); (e) in said housing 200 a: at least one means 202 a for focusing, towards the at least one sensor 202, the portion of the radiation irradiated by the radiation-emission means 201 which is reflected by the human's skin (cutis); (f) in said housing 200 a: at least one means 206 capable of fixing the position of said at least one radiation emission means 201, said at least one focusing means 202 a, said at least one sensor 202 and said at least one radiation diffusion means 205 relative to each other; and (g) at least one flat and rigid surface area 207 capable of allowing the passage of the radiation emitted from said at least one radiation emission means 201 out of said housing 200 a towards the human's skin (cutis) and of the radiation reflected from the human's skin (cutis) into said housing 200 a towards the at least one focusing means 202 a and the at least one sensor 202.

In a preferred embodiment of the invention (but the invention is not restricted to this embodiment), the device 200 according to the present invention for a measurement of the status, as far as relevant for determining the actinic irradiation dose, of the skin of a person and - particularly preferred - for a measurement of the skin color and/or of the skin status of a person, has the shape of an oblique truncated cone, but further conceivable embodiments will be described below in detail.

It may be mentioned here that the device 200 as far as the above features (a) to (c) are concerned, is very similar to the device for measuring the skin color and/or skin status as described in the Applicants' earlier patent application published as EP-A 1 508 301.

In other words: As the device according to the prior art document EP-A 1 508 301, the device 200, according to the present invention, for a measurement of the status and/or color, as far as relevant for determining the applicable actinic irradiation dose, of the skin of a human on his/her skin (cutis), comprises (a) at least one means 201 for emitting radiation having a defined spectral composition onto the skin (cutis); (b) at least one sensor 202 for measuring the portion of the radiation irradiated by the emission means 201 which is reflected by the skin (cutis) as well as (c) a means 212 for providing energy to the at least one emission means 201 and/or to the at least one sensor 202 and for transmitting measured data to a data analysing means 203.

In preferred embodiments of the invention, the means 201 for releasing or emitting, respectively, radiation having a defined composition may be one or more light emitting diodes (LEDs) or a light guide. The former may be used as one single LED or as groups of two, three, four or even more LEDs; in the case of two, three, four or even more LEDs, these LEDs may be (but not necessarily have to be) different with respect to the wavelength of the LEDs emitted light.

Alternatively, a light guide advances light of a defined spectral composition from a light source (which usually is spaced away from the emitting end of the guide). In accordance with the invention, the use of several (e. g. two, three, four or even more) LEDs is particularly preferred. In such a case, several LEDs are - even more preferably - different from each other with respect to the wavelength of the emitted light. In a particularly preferred embodiment according to the invention, three LEDs are used. The three LEDs of this particularly preferred, albeit not restricting, embodiment may emit, for example, blue, green and red light (which is, however, preferred but not compulsory and, hence, not restrictive, to the present invention).

The term "radiation having a defined spectral composition" as used in the specification and claims, is understood to mean, in the context of the present invention, radiation the wavelengths of which are selected from the continuous spectrum of the light, particularly preferred of the visible light, i.e. of the light having wavelengths of 400 to 800 nm, in such a way that the irradiated light can be reflected by the skin (cutis) and provides usable results of the measurements of the status of the skin, as far as relevant for determining the actinic radiation dose (e. g. the UV radiation does and/or the visible radiation dose and/or the IR radiation dose), and particularly, a measurement of the skin color and of the skin status, respectively. In the present invention, it is possible to use radiation having wavelengths extending over the whole spectrum, particularly radiation of the visible light spectrum, or to use monochromatic radiation. In particularly preferred embodiments of the invention, light of specific wavelengths is used, for example light of wavelengths in the range of from about 400 to 450 nm (blue), 470 to 520 nm (green) and 700 to 750 nm (red). Having in mind reproducible results, particularly of the measurements of the skin color and/or the skin status, the emission of light in the above-referenced three areas of wavelengths of the visible wavelengths areas is particularly approved.

The light of a defined spectral composition is provided by LEDs as the emission devices 201 in a further preferred embodiment of the invention. Those LEDs may be diminished in size so far that they may be accommodated as single or plural ones in the device 200 and, despite their small size, reliably provide the light of a defined spectral composition and emit it to the user's/human's skin.

In an alternative embodiment of the device 200 according to the invention, the light of a defined spectral composition later to be emitted to the skin (cutis) is generated at a place spaced away from the device. The light thus generated - possibly is a narrow or a broad spectral distribution of the wavelengths - is guided to the emission means 201 by one or more light guide(s) and is emitted to the skin from said device 201. In such a case, the device 200 for a measurement of the status and/or color, as far as relevant for determining the applicable actinic irradiation dose, of the skin of a human, or user, on his/her skin (cutis), as an optional feature, additionally comprises a means 211 for advancing the radiation of a defined composition to the emission means 201.

In accordance with the invention, the device 200 further comprises at least one sensor 202 serving a measurement of the radiation reflected by the skin (cutis) (or serving the measurement of the irradiation absorbed by the skin (cutis), in the sense of the equation: irradiated radiation minus reflected radiation is equal to absorbed radiation), based on the radiation irradiated by the emission device 201. There may be used one sensor 202, or there may be used several sensors 202. The use of only one sensor 202 is a preferred embodiment (although not restricting the invention). In other embodiments of the invention, there may be used one sensor 202 per emitted wavelength. For the above (non-restricting) example of three emitted wavelengths, a use of three sensors 202, i.e. one per emitted wavelength, may be appropriate; what may be decided by a skilled person in accordance with the conditions of a specific case.

As the at least sensor 202, any sensor or detector may be used which a person skilled in the area of measuring light in accordance with its wavelength and/or intensities knows. Usual photodiodes are preferred. As an alternative, photographic cameras (optionally miniaturized) in a form known to a skilled person may be used, for example a linear-array camera or optical camera. In particularly preferred embodiments of the invention, the sensor 202 may be used together with additional optical devices, for example with one or more prism(s) for splitting the reflected radiation into radiation distinguished by wavelengths in order to be in the position to detect radiation of one or more defined wavelength(s) in the light reflected from the skin (cutis).

The sensor 202 takes a measurement of the radiation reflected from the skin (cutis) dependent from the status relevant for determining the actinic irradiation dose for the skin of a person, e. g. the UV radiation dose and/or the visible light dose and/or the IR radiation dose. Particularly, it was found that, depending upon the color of the skin or, respectively, upon a pigmentation already occurred, for example, as a result of a previous irradiation of tanning UV radiation, different amounts of irradiated radiation having a defined spectral composition are reflected by the skin.

More in detail: White or not yet pre-pigmented or moderately pre-pigmented skin reflects more radiation than dark skin or skin already pre-pigmented. The dependency of the amount of reflected radiation, based on a defined amount of irradiated radiation having a defined spectral composition, allows an exact assessment of the irradiation dose and/or the irradiation time with a surprisingly high reliability. Particularly, the invention takes into account that different parts of a person's body have a different status of the skin, as far as relevant for determining the actinic irradiation dose, specifically have a different skin color and/or have a different skin status. This may, for example, result from the fact that every day a specific part of the body (for example the face or the arms) is/are exposed to actinic radiation as, for example, tanning UV radiation, more often than another part of the body (for example the chest or the back) and, hence, they have another skin color and another skin status, for example the skin tan status, respectively. Based on, for example, a pre-pigmentation previously conducted, the face and the arms may receive stronger actinic radiation doses, for example UV radiation doses, and/or may be irradiated for a longer time than the chest and the back. A measurement of the status, as far as relevant for determining the actinic irradiation dose, e.g. UV radiation dose, of the respective parts of the skin, for example, preferably a measurement of the skin color or the skin status, e.g. the skin tan status, respectively, by means of an emission of light having a defined spectral composition and the subsequent measurement of the amount of reflected light (reflected, on the one hand, by the pre-tanned and, hence, darker parts of the skin, of the face and of the arms and, on the other hand, by those parts of the skin not having received a tanning and, hence, being lighter, as for example, the skin of the chest or of the back) provides a surprisingly reliable standard for evaluating the UV irradiation dose which may be irradiated onto the skin or for a possible time of irradiating actinic radiation, for example UV radiation, having a defined intensity without exceeding the erythema threshold.

In accordance with the invention, the device 200 for a measurement of the status and/or color, as far as relevant for determining the applicable actinic irradiation dose, of the skin of a human on his/her skin (cutis), comprises at least means 212 for providing energy to the at least one emission means 201 and/or to the at least one sensor 202 and for transmitting measured data to a data analyzing means 203

The amount of reflected radiation determined by the at least sensor 202, preferably the one sensor 202, is transmitted via means 212 comprised by the device 200 to a data analysing means 203. It is the purpose of this means to provide, in addition to an analysis of the data received, information about the intensity and duration of a possible irradiation of certain parts of the body with actinic radiation, for example with UV radiation and/or with visible radiation and/or with IR radiation, and to thereby initiate a control for exposing certain parts of the body to less intensive actinic radiation (UV and/or visible and/or IR radiation) and to expose other parts of the body to stronger actinic radiation (e. g. tanning UV radiation and/or visible radiation and/or IR radiation). In accordance with the invention, the means 212 may also provide the necessary energy to the sensor.

The device 200 for a measurement of the status and/or color, as far as relevant for determining the applicable actinic irradiation dose, of the skin of a human on his/her skin (cutis) is further described in detail be referring to preferred embodiments of the device 200 as shown in the Figures 2 to 6.

In accordance with the present invention, the device 200 for a measurement of the status and/or color, as far as relevant for determining the applicable actinic irradiation dose, of the skin of a human on his/her skin (cutis), comprises, in said housing 200 a, at least one means 205 for diffusing the radiation, emitted by the radiation emission means 201, between the radiation emission means 201 and the human's skin (cutis)

Hence, in accordance with the present invention, the device 200, in the area of radiation emitted by the emission means 201, comprises said at least one device 205 serving the diffusion of the emitted light between the emission device 201 and the skin (cutis) of the human/user. By the at least one device serving the diffusion of the emitted light, the actinic radiation direction is changed from the single directed beam to a plurality of diffuse beams. Particularly, a sphere-shaped cavity 205 within the device 200 has proven advantageous, which cavity serves as a diffusor 205 and changes the direction of the emitted radiation between the emission device 201 and the user's skin (cutis).

The device 200 comprises at least one device 205 serving the diffusion of the emitted light between the emission device 201 and the skin (cutis) of the human/user. There may be present one device 205 for the diffusion of the emitted light, or there may be present several devices 205 for the diffusion of the emitted light, for example two, three or even four devices 205 for the diffusion of the emitted light. In a preferred embodiment of the invention which, however, does not restrict the invention, the device 200 for a measurement of the status and/or color, as far as relevant for determining the applicable actinic irradiation dose, of the skin of a human on his/her skin (cutis) comprises one device 205 serving the diffusion of the emitted light between the emission device 201 and the skin (cutis) of the human/user.

In a preferred embodiment of the invention, a so-called "Ulbricht sphere" is used as the diffusor 205. This is advantageous in generating a diffuse radiation very similar to the radiation of natural sunlight. For irradiating light for a measurement of the status, as far as relevant for determining the actinic irradiation dose, of the skin of a human person or user, this solution has the advantage that a measurement radiation is irradiated onto the skin (cutis) of the human or user which is very similar to the radiation received by the skin/cutis of the user in a natural environment.

In accordance with the invention, the device 200 for a measurement of the status and/or color, as far as relevant for determining the applicable actinic irradiation dose, of the skin of a human on his/her skin (cutis) comprises, in said housing 200 a, at least one means 202 a for focusing, towards the at least one sensor 202, the portion of the radiation irradiated by the radiation-emission means 201 which is reflected by the skin (cutis). It is particularly advantageous for the present invention that the light reflected by the user's skin (cutis) while being irradiated with (preferably) visible light is focused, on its way back into the housing 200 a and towards the sensor 202, by said at least one means 202 a for focusing the light, because a reproducible and - with respect to exactness - considerably improved measurement of the user's skin status(type and/or skin color is achieved.

As described above, the device 200 comprises at least one device 202 a serving the focusing of the reflected light between the user's skin (cutis) and the sensor 202. There may be present one means 202 a for focusing the reflected light, or there may be present several means 202 a for focusing the reflected light, for example two, three or even four means 202 a for focusing the reflected light. In a preferred embodiment of the invention which, however, does not restrict the invention, the device 200 for a measurement of the status and/or color, as far as relevant for determining the applicable actinic irradiation dose, of the user's skin on his/her skin (cutis) comprises one means 202 a serving focusing the reflected light on its "way" from the user's skin (cutis) and the sensor 202.

There may be used any means 202 a for focusing the light reflected by the user's skin which is conceivable by a skilled person for such a purpose, and each conceivable focusing means 202 a may be selected by a skilled person in accordance with the requirements. In accordance with a preferred embodiment of the invention, the focusing means 202 a is at least one lens or a combination of lenses, preferably one lens, more preferably one collecting lens, even more preferably a collecting lens having a focus which is adapted to the present device 200 in such a manner that the lens focus exactly meets the optical area of the sensor 202. In an exemplary embodiment, the focus of the focusing means 202 a, preferably of the lens 202 a, is selected in such a manner that it meets the optical area of the sensor, e. g. of a photodiode, in order to allow a focused collection of the radiation reflected from the user's skin (cutis) by the photodiode. In the case of such a preferred embodiment, the sensor 202, e. g. in the form of a photodiode, may exactly collect the radiation emitted from the user's skin (cutis) so as to enhance the exactness of the determination of the user's skin status/type and/or skin color.

In accordance with the invention, the device 200 for a measurement of the status and/or color, as far as relevant for determining the applicable actinic irradiation dose, of the skin of a human on his/her skin (cutis) comprises, in said housing 200 a, at least one means 206 capable of fixing the position of said at least one radiation emission means 201, said at least one focusing means 202 a, said at least one sensor 202 and said at least one radiation diffusion means 205 relative to each other.

Hence, the device 200 comprises, for example, at least one means 206 for adjusting defined distances between the at least one radiation emission means 201 and the skin (cutis), on the one hand, and between the skin (cutis), the at least one focusing means 202 a and the at least one sensor means 202, on the other hand. Also the at least one radiation diffusion means 205 has defined distances from the at least one radiation emission means 201, the at least one focusing means 202 a and the at least one sensor means 202. In an advantageous way, a fixed arrangement, for example in a measuring head or measuring sensor contributes to achieving such defined distances, as was already disclosed in the document EP-A 1 508 301. By such a measuring head or measuring sensor, a specific arrangement of the respective means in a housing 1 or 200a for improving the precision of the measurement and for facilitating the practical operation of the device 200 during a measurement is achieved.

In accordance with the invention, the device 200 for a measurement of the status and/or color, as far as relevant for determining the applicable actinic irradiation dose, of the skin of a human on his/her skin (cutis) comprises at least one means 206 capable of fixing the position of said at least one radiation emission means 201, said at least one focusing means 202 b, said at least one sensor 202 and said at least one radiation diffusion means 205 relative to each other.

The term "at least one means 206", as used in the present specification and claims, is understood to mean one means 206 or several means 206, for example two means 206, three means 206, four means 206 or even five or more means 206, although not restricting the present invention to such cases of two, three, four or five means 206. In preferred embodiments, the device 200 comprises one means 206 capable of fixing the position of said at least one radiation emission means 201, said at least one focusing means 202 a, said at least one sensor 202 and said at least one radiation diffusion means 205 relative to each other, or the device 200 comprises one means 206 per each device (i.e. radiation emission means 201, sensor 202 and radiation diffusion means 205) the position of which has to be fixed relative to any other of the means radiation emission means 201, focusing means 202 a, sensor 202 and radiation diffusion means 205. Usually, one means 206 capable of fixing the position of said at least one radiation emission means 201, said at least one focusing means 202 a, said at least one sensor 202 and said at least one radiation diffusion means 205 relative to each other, per each of said means (i.e. radiation emission means 201, focusing means 202 a, sensor 202 and radiation diffusion means 205) is used, i. e. three means 206 capable of fixing the position of said at least one radiation emission means 201, said at least one focusing means 202 a, said at least one sensor 202 and said at least one radiation diffusion means 205 relative to each other are used. In an even more preferred embodiment of the device 200 of the invention, one means 206 capable of commonly fixing the position of said at least one radiation emission means 201, said at least one focusing means 202 a, said at least one sensor 202 and said at least one radiation diffusion means 205 relative to each other is used.

In preferred embodiments of the present invention, such means 206 capable of fixing the position of said at least one radiation emission means 201, said at least one focusing means 202 a, said at least one sensor 202 and said at least one radiation diffusion means 205 relative to each other are usual fixation means which a skilled person knows for such fixation purposes and which a skilled person may select, based on technical experience, in accordance with the requirements of a specific case. Examples (which do not restrict the present invention) are holders or clamps or similar fixation means.

In a preferred embodiment of the device 200 of the invention, the means 206 capable of fixing the position of said at least one radiation emission means 201, said at least one focusing means 202 a, said at least one sensor 202 and said at least one radiation diffusion means 205 relative to each other is one fixation means 206, which brings all four parts (i) the radiation emission means 201 (preferably the one, two or three LED(s) emitting suitable radiation, preferably in the range of wavelengths in the visible range); (ii) the focusing means 202 a (preferably the collecting lens 202 a), (iii) the sensor 202 (preferably the photodiode 202) and (iv) the radiation diffusion means 205 (preferably the Ulbricht sphere 205) into a fixed proximity to each other.

Preferably, said fixation means may be selected from parts made of plastics or made of a metal or of a metal alloy, more preferably made of plastics. A skilled person well knows that such parts may easily be made of a plastics material, i. e. of a polymer, for example of a polycarbonate polymer, by a mold injection process so as to arrive at a relatively hard, rigid, shock-resistant plastics part which may be brought into close adhesion to the above four components. The result may, for example, be seen from Figures 4 and 5 as well as from Figures 7 A to 7 C.

When using such a polymer part 206, for example a polycarbonate polymer part (see Figures 4 and 5), the radiation emission means 201, e. g. a series of LED's 201, is accommodated on the "upper" side of said part 206, i. e. on the side opposite to the measuring surface area 207, in a type of "pocket" fixing its position relative to the other components mentioned above.

In a similar manner, the radiation diffusion means 205, e. g. the Ulbricht sphere 205 (which may consist of two composable semi-sheres, the "lower" one of which is shown in Figure 4 at "205 a", which are connected and fixed to each other by a type of "O-ring" shown in Figure 5 at "205 b"), is inserted, at its lower semi-sphere into a receiving area, for example a receiving ring (shown in Figure 4 at "206 a"), thereby fixing the lower semi-sphere 205 a (releasably, but fixed for, and during the time of, the operation of the device 200) to the fixing means 206. In preferred embodiments of the invention, the Ulbricht sphere 205 (particularly its lower semi-sphere 205 a) is fixed to the fixing means 206 by means of a so-called latch or snap-fit, preferably at "206 a" in Figure 4 on the lower outer side of the lower semi-sphere 205 a and at the lower inner side of the plastics polymer part 206 at "206 a" in Figure 4.

Moreover, the combination of the lens 202 a (as the focusing means 202 a) and of the sensor 202, e. g. the photodiode 202, are fixed to the plastics polymer part 206 on the "upper" side opposite to the measuring surface area 207, in a type of "pocket" fixing the position of the lens (focusing means 202 a) and of the photodiode (sensor 202) relative to the other components mentioned above.

As described above, the four components (i) radiation emission means 201 (preferably the one, two or three LED(s) emitting suitable radiation, preferably in the range of wavelengths in the visible range); (ii) focusing means 202 a (preferably the collecting lens 202 a), (iii) sensor 202 (preferably the photodiode 202) and (iv) radiation diffusion means 205 (preferably the Ulbricht sphere 205) are fixed, relative to each other. By such a fixation via the plastics part 206 as the means capable of fixing the position of the components to each other, the four components providing and measuring the radiation for a determination of the status and/or color of the user's skin (cutis) have fixed distances to each other. By such fixed distances, an exact and reproducible determination of the user's skin status/type and/or of the user's skin color may be effected, and no measuring errors due to a movement of the above components relative to each other can occur. In addition, the distances and angles between the four components cannot be changed and remain constant

In the following, it will be described in detail how a fifth component, i. e. at least one flat and rigid surface area 207 for the measurement is included into the above-described fixed arrangement of the above-mentioned four components. In accordance with the present invention, the device 200 for a measurement of the status and/or color, as far as relevant for determining the applicable actinic irradiation dose, of the skin of a human on his/her skin (cutis) further comprises at least one flat and rigid surface area 207 capable of allowing the passage of the radiation emitted from said at least one radiation emission means 201 out of said housing 200a towards the human's skin (cutis) and of the radiation reflected from the human's skin (cutis) into said housing 200a towards the at least one focusing means 202 a and the at least one sensor 202.

There may be comprised, by said device 200, one such flat and rigid surface area 207, or there may be comprised several such flat and rigid surface areas 207, for example two, three or even four flat and rigid surface areas 207. The case of one flat and rigid surface area 207 comprised by the device 200 is, by far, preferred in order to achieve a reliable measurement of the user's skin's (cutis) status and/or of the user's skin color for the subsequent electronic control of actinic radiation irradiated onto the user's skin (cutis).

The shape of the at least one (or of the one, of the two, of the three, etc.) surface area(s) 207 is not restricted for the present invention and may be freely selected by a skilled person in accordance with the experience in shapes of such parts and with the requirements of the specific case. In view of the purpose of said surface area 207, i. e. to have available a flat two-dimensional plane allowing a full contact between the user's skin and the measurement device 200, the shape may preferably be selected from the group consisting of a round (or circular) shape and an oval shape and a diamond (or rhombus) shape and a rectangular shape and even a square shape. For obvious reasons, a round (or circular) shape or an oval shape is preferred, since it well fits with the user's skin surface even in areas of skin condition or skin color measurement which are relatively small or narrow in diameter (e. g. the skin of the face). In addition, it may well be included into the fixed arrangement of the other four components into the plastics polymer part 206 described above.

The size of the at least one (or of the one, of the two, of the three, etc.) surface area(s) 207 is not restricted for the present invention and may be freely selected by a skilled person in accordance with the experience in sizes of such parts and with the requirements of the specific case. For a rectangular or diamond shape flat and rigid surface area, the rim lengths may be in a range of from 10 mm to 50 mm, preferably in the range of from 15 mm to 30 mm. Preferred sizes for a square shape or diamond shape surface area may be, for example and without restriction, 5 mm x 5 mm or 10 mm x 10 mm or 10 mm x 15 mm or 15 mm x 25 mm. For a round (or circular) or oval shape flat and rigid surface area, the diameters may be in a range of from 5 mm to 20 mm, preferably in the range of from 8 mm to 15 mm, for example. Preferred sizes for a round shape or an oval shape surface area may be, for example and without restriction, 10 mm, 11 mm, 12 mm, 13 mm, 14 mm or 15 mm (round) or 10 mm x 15 mm (oval).

The material of the at least one (or of the one, of the two, of the three, etc.) surface area(s) 207 is not restricted for the present invention and may be freely selected by a skilled person in accordance with the experience in materials and with the requirements of the specific case. In the present specification and claims, the terms "flat" and "rigid" are understood to mean parts for said surface area(s) 207 which are neither bent nor bendable and form (round, oval, square etc.) parts of a planar shape. In addition, the materials (as described below) are such that the form cannot be changed into a bent shape due to a flexibility of the material, because the material is rigid and does not allow bending.

In view of the purpose of said surface area 207, i. e. to have available a flat two-dimensional plane allowing a passage of radiation of specific wavelengths on its way from the at least one radiation-emitting means 201 (optionally via the diffusion means) in the housing 200a to the human's/user's skin (cutis) and a passage of the radiation reflected by the human's/ user's skin (cutis) to the at least one sensor 202 in the housing 200a, the material of the at least one flat and rigid surface area 207 may preferably be selected from optical glasses, preferably from optical glasses of a polished quality. In a preferred embodiment which is suited to ensure good results of the measurement for the determination of the user's skin status/type and/or skin color, the material of the surface area(s) 207, preferably of the one surface area 207, is a polished glass having high transparency for those wavelengths of the radiation emitted by the radiation-emitting means 201 and reflected by the user's skin (cutis). An even more preferred material for the at least one surface area 207 is selected from the group consisting of polished glasses having a high transparency for the wavelengths of from about 400 to 450 nm (blue), 470 to 520 nm (green) and/or 700 to 750 nm (red). As mentioned above, radiation of, for example, these wavelengths may suitably be used for the determination of the user's skin status/type and/or skin color in order to obtain ideal results of the user's skin status/type and/or skin color determination measurements of the reflected radiation.

In accordance with the invention, the at least one flat and rigid surface area 207 capable of allowing the passage of the radiation emitted from said at least one radiation emission means 201 out of said housing 200 a towards the human's skin (cutis) and of the radiation reflected from the human's skin (cutis) into said housing 200 a towards the at least one focusing means 202 a and the at least one sensor 202 is arranged in such a way that it forms a type of end wall of the housing 200 a directed towards the user's skin (cutis) the color and/or status of which is to be determined. As such, it may be fixed, in a conventional manner (e. g. adhered by gluing or by being formed integrally in connection to the housing) to the housing 200a so as to be fixed permanently to the housing 200a.

According to the invention, which result into an improved suitability of the device 200 for the purpose of a measurement of the status and/or color, as far as relevant for determining the applicable actinic irradiation dose, of the human's skin on his/her skin (cutis), the device 200 further comprises a flexible membrane 208 which incorporates, said flat and rigid surface area 207, more preferably which comprises said flexible membrane 208 in a releasable connection to said housing 200 a. In this case, and in contrast to the embodiment described above, said flat and rigid surface area 207 may be held by said membrane 208 housed or attached, for example releasably attached, or even fixedly incorporated, in such a manner that either the membrane 208 and the surface 207 may be separated from each other or, alternatively may be fixed to each other and cannot be separated. The resulting combination of membrane 208 and surface 207 (either separatable or permanently combined) may be fixed to the housing 200a in a permanent manner or, in a preferred embodiment of the invention, may be detachably or releasably attached to the housing 200a in order to be removed for cleaning and/or in order to be replaced, when damaged in any way.

In the present specification and claims, the term "the membrane 208 houses the surface 207 " is understood to mean a connection between the membrane 208 and the flat and rigid surface 207 which may be detached: The surface 207 is releasably connected to the membrane. In contrast, in the present specification and claims, the term "the membrane 208 incorporates the surface 207" is understood to mean a connection between the membrane 208 and the surface 207 which is intended to be a permanent connection from which the surface cannot be released without destroying the membrane and/or the surface.

In particularly preferred embodiments of the invention, the device 200 comprises the membrane 208, together with the surface 207, in such a way that the membrane 208/surface 207 combination may be released from the connection to the housing 200a. Such a fixation may be effected by any suitable fixation a skilled person knows for making a releasable connection between the housing 200a and the membrane 208, for example by screwing the membrane 208 onto a corresponding thread at the end of the housing 200a or by connecting the membrane 208 and the housing 200a by a bayonet fitting, without restricting the invention to those. This is particularly preferred because the membrane 208/surface 207 combination may easily be removed, particularly easily with a suitable tool, from the housing. Releasing the membrane 208/surface area 207 combination from the housing 200a may occur either for cleaning, and hence for hygienic purposes, in view of the fact that the surface area 207 (and also the membrane 208) are contacted by the user's skin in any case if, and each time when, the user's skin color and/or skin status is determined. Another reason is that the membrane 208 (and optionally also the surface area 207) may need to be exchanged in certain service intervals in order to prevent them from being deteriorated and from becoming unsuitable for their intended purpose.

In particularly preferred embodiments of the invention, the connection between the membrane 208 and the housing 200a, on the one hand, and the connection between the membrane 208 and the surface area 207 is made by inserting the material of the flexible membrane 208 into a groove or notch available at the lower (in the position of using the device 200) end of the housing 200 a, on the one hand, and into a groove or notch available around rim or fitting of the surface area 207 at the injection molding plastics part 206 effecting a mutual fixation of the surface area 207 and the other optical components of the device 200 relative to each other. In other words: In the course of manufacturing the connection between the relevant arts, there is made a injection molding two components part comprising the injection molding plastics part 206, on the one hand, and the material of the flexible membrane 208, on the other hand, which two parts are connected directly to each other by injection molding. As will be described below, such a connection allows the unit comprising the injection molding plastics part 206, the radiation-emitting means 201, for example the LED's 201, the diffusing means 205, for example the Ulbricht sphere 205, the surface area 207 and the sensor 202 plus the focusing means 202 a, for example the photodiode 202 plus the lens 202 a, to be adapted for a measurement of the user's skin status/type or skin color with respect to every conceivable angle relevant during the measurement, relative to the user's skin surface, and an exact measurement of the user's skin status/type and/or skin color can reliably be achieved.

The flexible membrane 208, in even more preferred embodiments of the invention, comprises or even consists of a material which allows, during use, the membrane 208 to be free to move into all directions to a certain extent, while being fixed to the end to the housing 200a and to the injection molding plastics part 206, but allowing the surface area 207 housed or incorporated in the membrane 208 to movingly or tiltingly adapt to the user's skin surface during the intended measurement of the user's skin color and skin status, in order to effect a precise measurement of the user's skin color and/or skin status. If the surface 207 can be put into a position fitting with (e. g. being exactly parallel to) the user's skin surface precisely due to the flexibility of the membrane 208, a reliable determination of the user's skin color and/or skin status can be achieved.

In preferred embodiments of the invention, the flexible membrane 208 is made of a material selected from the group consisting of any desirable material having flexibility, long term durability, being resistant to the actinic radiation used in the apparatus wherein the device 200 is employed, and being resistant to solvents eventually used for cleaning the device 200 or single components thereof. Preferred materials are organic pliable polymers, and particularly preferred are silicones. Particularly if a preferred silicone material used as the membrane material, the desired flexibility of the membrane 208, on the one hand, and the essential durability of the membrane 208 and injection moldability to the hard plastics material (e. g. to a polycarbonate), on the other hand, are achieved so that the membrane-housed or, preferably: membrane-incorporated, surface area 207 can be brought into a tight, preferably parallel, contact to the user's skin surface, where the desired measurement of the skin status and/or skin color is to be effected.

In even more preferred embodiments of the device 200 of the invention for a measurement of the status and/or color, as far as relevant for determining the applicable actinic irradiation dose, of the skin of a human on his/her skin (cutis), said releasable connection of the flexible membrane 208 to the housing 200a and to the rim/fitting around the surface area 207 is sealed to be impermeable for gases, vapours, liquids and solids. This is a particularly advantageous embodiment of the invention, because the releasable connection between the membrane 208 and the housing 200a of the device cannot be deteriorated neither by solvents (and their vapours) used for cleaning nor by sweat and/or fat from the user's skin surface (coming into contact to the surface 207 and/or to the membrane 208 repeatedly) nor by any other particles of dust and other contamination. In this way, also the inner space of the housing 200a is prevented from being influenced by such contaminations in an advantageous manner.

The sealing in the area of the releasable connection of the flexible membrane 208 to the housing 200a and to the rim/fitting around the surface area 207 is effected in a manner well-known to a skilled person, who may select suitable sealing materials and methods based on his experience and in accordance with the specific requirements of the case without being imposed with any restriction. In particularly preferred embodiments of the invention, the sealing is effected by a suitable sealing material selected from organic, more specifically polymeric organic sealing materials as, for example, silicones, which are commonly used for sealing purposes. In an even more preferred embodiment, the silicone material of the membrane is connected to the housing and/or to the injection molding plastics part 206 in the course of the injection molding process directly, for example (as mentioned above) by injection molding the pliable silicone material into a groove or notch of the hard plastics material. Most advantageously, such a preferred sealing completely prevents gases, vapours, liquids and solids from deteriorating the connection of the flexible membrane 208 to the housing 200a and to the rim/fitting around the surface area 207 of the device 200 of the invention.

A further preferred embodiment of the invention is directed to a device 200, which further comprises at least one means 209 capable of actuating the human's skin status and/or skin color measurement. As will be described below in the specification, when placing the device 200 with its flat and rigid surface area 207 onto the user's skin for effecting a measurement of the status and/or color, as far as relevant for determining the applicable actinic irradiation dose, of the skin of a human/ user on his/her skin (cutis), the measurement must be actuated at a time when the surface area 207 is suitably fitted on the user's skin, preferably when the surface area 207 is completely parallel to the user's skin surface.

There may be comprised, by said device 200, one means 209 capable of actuating the human's skin status and/or skin color measurement, or there may be comprised several means 209 capable of actuating the human's skin status and/or skin color measurement, for example two, three or even four means 209 capable of actuating the human's skin status and/or skin color measurement. The case of one means 209 capable of actuating the human's skin status and/or skin color measurement comprised by the device 200 is preferred in order to achieve a reliable measurement of the user's skin's (cutis) status for the subsequent electronic control of actinic radiation irradiated onto the user's skin (cutis). Moreover, one such means 209 capable of actuating the measurement is sufficient and, in addition, needs no reservation of free space within the housing for similar actuating means having the same function as the first one.

Generally, said at least one means 209, preferably said one means 209, capable of actuating the human's skin status and/or skin color measurement may be each conceivable means 209 capable of actuating the human's skin status and/or skin color measurement a skilled person may consider, based on the general experience in this field, and in accordance with the requirements of the specific case. Exemplary (but not restricting) examples of such means 209 capable of actuating the human's skin status and/or skin color measurement are switches, e. g. press-sensitive switches either in the area where the device 200 contacts the user's skin surface (as in the corresponding device of the prior art document EP-A 1 508 301 where, upon the contact pressure's exceeding a certain value, the pressure-sensitive contact switch actuates the human's skin status and/or skin color measurement) or in the area where the user grasps the device 200 with his/her hand and, at a certain moment of time, manually actuates the human's skin status and/or skin color measurement. Of course, other means 209 capable of actuating the human's skin status and/or skin color measurement may also be used.

In a preferred embodiment of the invention, the device 200 comprises at least one, preferably one, actuating means 209 which is disposed within the housing 200 a. This is a preferred embodiment in view of the fact that the means 209 capable of actuating the human's skin status and/or skin color measurement is protected within the housing 200a and may also be arranged close to the electronic circuits on which it will act upon actuation by the actuating means 209.

Even more preferred are embodiments of the invention, wherein said at least one actuating means 209, more preferably: the one actuating means 209, is disposed within the housing 200a on a side of the radiation diffusing means 205, which is opposite to the side of the flat and rigid surface area 207. This may be suitably explained by referring to Figures 2, 3, and 6.

In Figures 2 and 3 (which show the same device 200 for a measurement of the status and/or color, as far as relevant for determining the applicable actinic irradiation dose, of the skin of a human on his/her skin (cutis)), the housing 200a encompasses or houses all means which enable the human or user to carry out said measurement of the status and/or color, as far as relevant for determining the applicable actinic irradiation dose, of the skin of a human. Energy is provided to the housing 200a via the means 212 for providing energy to the at least one (preferably one) emission means 201 and/or to the at least one (preferably one) sensor 202. Data measured by the device 200 (as described below) may be transmitted to a data analyzing means or directly to energy control means also via lined guided through said means 212. Alternatively, a data transmission may also be effected via other routes, e. g. via suitable wireless transmission; in the latter case, the at least one means 212 is still used for the other purposes mentioned above.

In the bottom part of the housing 200a, the at least one (preferably the one) flat and rigid surface area 207 forms the lower closure of the housing 200a. As described above, said flat and rigid surface area 207 is incorporated, into the flexible membrane 208. The flexible membrane 208, on the side opposite to the side where the surface area 207 is housed or incorporated, is fixed to the lower end of the housing 200a and, in even more preferred embodiments even sealingly connected to the lower end of the housing 200a in a manner completely tight against gases, vapors and solids.

The means 205 for diffusing the radiation emitted by the emission means 201, optionally comprising the Ulbricht sphere, together with the at least one means 201 for emitting radiation of a defined spectral composition (or emission means 201) and the at least one sensor 202, optionally with the focusing means 202 a, together with the means 206 of the invention capable of fixing the position of the (at least one) radiation emission means 201, the (at least one) sensor 202 plus the (at least one) focusing means 202 a, and the (at least one) radiation diffusion means (e. g. the Ulbricht sphere) relative to each other are accommodated in the optical unit 220 (which is shown as a closed optical unit 220 (as in operation) in Figures 2, 3, 5 and 6 and is shown with removed upper semi-sphere for making visible the sensor 202, the focusing means 202 a and the fixation means 206 in Figure 4.

In a particularly preferred embodiment of the invention, which is shown in Figures 2 and 3 as well as in Figure 6, the device 200 comprises said at least one actuating means 209 within the housing 200 a and disposed on a side of the radiation diffusing means 205, which is opposite to the flat and rigid surface area 207 and is disposed centrally on the central longitudinal axis (A) of the housing 200 a. Said longitudinal axis (A) of the housing may be seen from Figures 2, 3 and 6, and the central place of disposition of the actuating means 209 is shown in Figures 2, 3 and 6, too. The central disposition of the at least one (preferably one) actuating means 209 is advantageous (and, hence, particularly preferred), because the actuating means 209 is disposed adjacent to other means in the housing 200a needed for the actuation and, hence, may easily cooperate with them, if the actuation of the measurement is to be effected.

The actuating means 209 disposed within the housing 200a of the device 200 may be any suitable actuating means a skilled person knows, on the basis of his general knowledge and in accordance with the requirements of this specific case, for actuating the measurement of the status and/or color, as far as relevant for determining the applicable actinic irradiation dose, of the skin of a human on his/her skin (cutis) in preparation for proceedings for irradiating actinic radiation onto the user's skin (cutis). The invention is not at all restricted with respect to such actuating means 209, as long as the effect a reliable actuation of the measurement with easy handling steps and find their space within the rigid housing 200a at the place defined above. In even more particularly preferred embodiments of the invention, the actuating means is a Hall sensor 209. Hall sensors are known to vary the transducer's output voltage in response to a magnetic field and are known as proximity switches, i. e. switches acting in reaction of changes of a magnetic field.

In a further preferred embodiment of the invention, the device 200 additionally comprises, preferably within the housing 200a, at least one magnet 210 capable of effecting a contact for actuating said actuating means 209. There may be present one magnet 210, or there may be present more than one magnet 210, for example two magnets, three magnets, four magnets or even five or more magnets 210. In a preferred embodiment of the invention, the magnet 210 or the plurality of magnets 210 is accommodated on the side of the Hall sensor 209 remote from the surface area 207. This may be seen in Figures 2, 3 and 6. The device 200 of the invention comprising one magnet is particularly preferred.

In particularly preferred embodiments of the invention, the device 200 comprises one magnet 210 having a strength to develop a sufficiently strong magnetic field to allow the Hall sensor 209 to be switched when approaching the magnet(s) by a certain threshold distance (or closer) small enough to effect on the Hall sensor 209. This will be explained in more detail below.

As the magnets 210, each conceivable magnet a skilled person knows for making contact in or for switches may be used. Particularly preferred are magnets in the form of common permanent magnets adapted generating a magnetic field in a closely adjacent area around the magnet and having a relatively high magnetic flux densities. Magnets measuring a high magnetic flux density at distances < 1 mm, which are preferably suitable for the operation of Hall sensors as described herein are commercially available, are known to a skilled person and may be selected in accordance with the requirements of the case. One specific example of a preferred magnet useable in combination with the above Hall sensor (without restricting the invention to such a magnet) is a rare earth element magnet (e. g. a neodymium magnet, exemplarily known as NdFeB magnet, Neodymium Iron Boron (NIB) magnet or NEO magnet), which is a typically useable permanent magnet capable of effecting a magnetic flux density measurement, at distances of, for example, 0.6 mm, of 3500 G (Gauss). With magnets of such a type, specifically with the above exemplary magnet, the function of switching the Hall sensor 209 approaching the magnet by a distance from, for example, > 1 mm (no switching) to e. g. 0.6 mm (switching) may be effected reliably, and the measurement of the status and/or color, as far as relevant for determining the applicable actinic irradiation dose, of the skin of a human on his/her skin (cutis) may be actuated easily, reliably and with excellent results over longer function times. This is described in detail below.

In the preferred embodiment of the present invention, the use of the combination of at least one Hall sensor 209, more preferably the one Hall sensor 209, and the at least one magnet 210, more preferably the one magnet 210, is as follows:

As may be seen from Figures 2 to 6 and 7 A to 7 C, the means making the present invention reliably functioning, i. e. (from the bottom of the device 200, and mentioning only one of these means, even if, in certain embodiments of the invention and as described in detail above, several (e. g. two, three or even more) of the one or the other or of all of these means may be used)
- the flat and rigid surface area 207 capable of allowing the passage of radiation in both directions, i. e. from the radiation emission means 201 (e. g. LED's 201) via the diffusing means 205 (e. g. Ulbricht sphere 205) through the flat and rigid surface area 207 (e. g. measurement window 207) to the user's skin and the radiation reflected from the user's skin through the flat and rigid surface area 207 (e. g. measurement window 207) via the diffusion means 205 (e. g. Ulbricht sphere 205) to the focusing means 202 a (e. g. lens 202 a) to the sensor means 202 (e. g. photodiode 202);
- the means 206 capable of fixing the mutual position of different means selected from radiation emission means 201 (e. g. LED's 201), diffusion means 205 (e. g. Ulbricht sphere 205), flat and rigid surface area 207 (e. g. measurement window 207), focusing means 202 a (e. g. lens 202 a) and sensor means 202 (e. g. photodiode 202), i. e. the (e. g.) injection-molded rigid plastics polymer part 206;
- the diffusion means 205 (e. g. the Ulbricht sphere 205);
- the radiation emission means 201 (e. g. the - preferably three - LED's 201);
- the focusing means 202 a (e. g. the lens 202 a);
- the sensor means 202 (e. g. the photodiode 202)
form one arrangement wherein the relative distances of all parts involved are fixed via the (e. g.) injection-molded rigid plastics part 206 capable of (and actually effecting) fixing of all parts mentioned above in a fixed and unchangeable position relative to each other.

In addition, the Hall sensor 209 is placed in a fixed position on top (in the position of the device 200 suitable and ready for operation) of the electro-optical measuring sensor unit 220, as may be seen from Figures 7 A to 7 C, as well as from Figure 3.

The whole unit described above is connected, via the sealing effected by injection molding in the surroundings of the flat and rigid surface area 207, to the flexible membrane 208, the outer rim of which is, in turn connected (e. g. via injection molding) to the housing 200 a of the device 200 of the invention.

As soon as, in the course of an actual measurement of the status/type and/or color of the user's skin by means of the device 200 of the invention, the flat and rigid surface area 207 (e. g. the measurement window 207) is brought in contact to the user's skin, the whole unit may move to an extent of a certain angle (relative to the axis A of the device 200) due to the fact that the membrane 208 is flexible, in order to seek the ideal measurement position. In the course of such movements, the whole unit (including the rigid part 206, the measurement window 207, the Ulbricht sphere 205, the LED's 201, the focusing lens 202 a and the photodiode/sensor 202 as well as the Hall sensor 209 as the actuating means 209) may follow the same movement, when seeking the ideal measurement position. Such an ideal measurement position is found as soon as the measurement window 207 (i. e. the flat and rigid surface area 207) has the correct position relative to the user's skin, e. g. a position completely parallel to the "lower") measurement window 207.

The magnet(s) 210, preferably the one magnet 210, positioned "above" the Hall sensor 209 is arranged in the housing 200 a of the device 200 in such a position that its (or their) magnetic field extends into such an area that, at the ideal measurement position of the unit containing all the other parts described above and contributing to the measurement, the Hall sensor(s) 209 has/have been moved into the area of the existing magnetic field generated by the magnet(s) 210. As a consequence of the Hall sensor's 209 having approached the magnet(s) 210 closer than the threshold distance of the magnetic field, the Hall sensor(s) 209 is/are "switched" from "OFF" to "ON" and start(s) the measurement by initiating the radiation emission means 201 (e. g. the LED's 201) to emit radiation towards the diffusing means 205 (e. g. the Ulbricht sphere 205), where the radiation is made diffuse. The radiation is proceeding towards the walls of the Ulbricht sphere 205 and, inter alia, to the area where the flat and rigid surface area 207 is positioned in a fixed manner. The radiation passes the measurement window 207, impinges onto the user's skin, is (in part) reflected (in part) back by the user's skin through the measurement window 207 and proceeds towards the focusing means 202 a (e. g. the lens 202 a) and to the sensor 202 (e. g. the photodiode 202) where its amount and wavelength range is measured.

The sensor 202 sends the signal immediately to the means controlling the power and intensity of the actinic radiation sources, thereby controlling which, and how much, actinic radiation of a certain wavelength may be irradiated onto the user's skin, and also for what time or irradiation.

Hence, a Hall sensor used as the actuating means 209, together with a magnet or magnets 210, is an advantageous embodiment of the invention, because a sensitive actuation of the measurement of the user's skin status/tape and/or color may be effected. Particularly, due to the spatially fixed arrangement of all means contributing to the measurement, the measurement is actuated only at a time when all parts contributing to the measurement are in the correct (aligned) position. No mis-measurement will occur due to this arrangement.

In accordance with the invention, the housing 200 a of the device 200 may have any suitable shape a skilled person may conceive for making the device suitable to perform the functions for which it is provided. Hence, examples of the shape of the device's housing 200 a are a cylinder, a cone, a cuboid, a sphere, to mention only several possible regular shapes of the device 200. Of course, the housing 200 a of the device 200 of the invention may also have irregular shapes, provided that the means needed for effecting the measurement of the status and/or color, as far as relevant for determining the applicable actinic irradiation dose, of the skin of a human on his/her skin (cutis), find a sufficient space within the housing, and the housing can be handled by the user in performing the desired measurement, in preferred embodiments of the invention: provided that the housing and its components housed therein can be handled by the user by employing one hand. In a preferred embodiment which is also shown in Figures 2, 3 and 6, the housing is rigid and has the shape of a cone or a truncated cone, more preferably of a truncated cone having an oblique axis A, with respect to the lower bottom of the truncated cone which corresponds to the surface area 207 for the measurement.

In accordance with the invention, the housing 200 a of the device 200 may have any suitable size a skilled person may conceive for making the device suitable to perform the functions for which it is provided. It is, of course, preferably that the size be as small as possible in order to allow an easy and convenient handling by the user. In addition, the housing 200 a of the device 200 has preferably such a size that it can be accommodated within the apparatus 100 for irradiating actinic radiation onto the user's body (as described below) after the measurement of the status and/or color, as far as relevant for determining the applicable actinic irradiation dose, of the user's skin on his/her skin (cutis). In view of the technical possibilities to miniaturize the above-mentioned (and further) components contained in the housing 200 a, a small size is not really a problem. Exemplary sizes (without restriction) are a length of from 5 to 20 cm, preferably from 10 to 18 cm, for example of 15 mm, along the axis A (see Figures 2, 3 and 6, if a cylinder or (truncated) cone is used) or along the longer axis (if a cuboid is used), and a diameter of from 3 to 8 cm, preferably of from 5 to 8 cm, (see Figures 2, 3 and 6, if a cylinder or (truncated) cone is used) or a length of the shorter axis of from 3 to 8 cm, preferably of from 5 to 8 cm (if a cuboid is used).

The material of the housing 200 a may be selected by a skilled person, based on usual experience and in accordance with the requirements of a specific case, from a plurality of known materials. Preferred requirements are the rigidity of the material so as to suitably protect the components housed therein from being damaged in the course of handling the device 200. On the other hand, the material should allow cleaning the device outer surface 200 with usual detergents, disinfectants and disinfecting detergent solutions and/or sprays. Moreover, the material should be resistant to the radiation used for the irradiation of the user's body. Preferred materials are plastic materials, i. e. polymers particularly preferably selected from the group consisting of rigid polymers; the preferred material for the housing 200 a of the device 200 of the invention is a polycarbonate; the invention is, however, not restricted to polycarbonates as the material for the housing 200 a.

In accordance with the invention, the housing 200a of the device 200 for a measurement of the status and/or color, as far as relevant for determining the applicable actinic irradiation dose, of the skin of a human on his/her skin (cutis) is suitable, with respect to shape, size and functional components contained therein, to be grasped by the user, preferably with one hand only, in order to carry out said measurement of the status and/or color, as far as relevant for determining the applicable actinic irradiation dose, of the user's skin on his/her skin (cutis).

In view of the fact that the user may have coated the hand (and body or at least parts thereof) with sweat and/or sunscreen (crèmes or lotions or emulsions) which may make the handling slightly difficult, the device's surface (i. e. the outer wall of the housing 200a of the device 200) may have grip areas being adapted to grasping the device 200 with a "slippery" hand. Such areas may have, for example, small grooves and/or projections or may even have recesses which may be grasped by the user's hand.

Grasping the device 200 appropriately by the user in the course of the measurement of the status and/or color, as far as relevant for determining the applicable actinic irradiation dose, of the user's skin on his/her skin (cutis) may be an important precondition for a correct and suitable measurement result. Hence, it may even be desired in a preferred embodiment of the invention that those areas of the housing surface which should ideally be grasped and appropriately hold by the user when effecting the measurement are specifically marked on the outer side of the housing 200a of the device 200. In a preferred embodiment, the housing 200a of the device 200 has one or more than one colored grip area(s) 230 showing to the user where to grasp and hold the housing, when effecting the measurement. There may be present one such colored grip area, or there may be present two or more, for example three or four, such colored grip areas 230. One specific embodiment of such colored grip area(s) is shown in Figure 1: The (in the exemplary case dark, e. g. blue) colored grip area 230 extends perpendicularly to the device's longitudinal axis and allows the user to easily recognize how to hold the device in the course of an appropriate measurement of the status and/or color, as far as relevant for determining the applicable actinic irradiation dose, of the user's skin on his/her skin (cutis), so as to bring the measuring surface area 207, controlled by the flexible membrane 208, in perfectly parallel alignment to the user's skin. Further colored grip areas may be present.

In an even more preferred embodiment of the invention, the colored grip area(s) 230 of the housing 200a of the device 200 is/are made of a material which allows passing colored illumination light from the inner side of the housing 200 a towards the outer side so as to be perceived by the user. This illumination light may direct the user's attention to that area/those areas of the device's outer surface, where the hands may grasp the device 200, for example by flashing light repeatedly flashing as soon as the user approaches the apparatus 100 for irradiating actinic radiation onto the user's body for receiving such irradiation. This feature may, for example, serve to reminding the user that the measurement of the status and/or color, as far as relevant for determining the applicable actinic irradiation dose, of the user's skin on his/her skin (cutis) is a mandatory safety requirement to be performed before the irradiation of the actinic radiation can be started.

In an even more preferred embodiment of the device 200 of the invention, the grip area(s) 230 is/are made of a material capable of allowing colored illumination light to pass from the inside, when addressed by electronic signals relating to measuring functions, towards the outer side, thereby signalling measuring functions to the user. This is an embodiment of additional advantage to the user: After taking (as will be described in detail below in the process section of this application) the device 200 by the one or several colored grip area(s) 230, directing the lower end of said device 200 housing the measurement surface area 207 together with the flexible membrane 208 to different areas of his/her skin (cutis), bringing said measurement surface area 207 in perfect parallel alignment to the skin surface areas and actuating the measurement(s) of the status and/or color, as far as relevant for determining the applicable actinic irradiation dose, of the user's skin on his/her skin (cutis) by means of the Hall sensor 209/magnet 210 combination, a valid measurement of the status and/or color, as far as relevant for determining the applicable actinic irradiation dose, of the user's skin on his/her skin (cutis) may be signaled to the user by a change of the color of the grip area(s) 230 due to addressing said grip area illumination by suitable electronic signals relating to the measurement functions, and thereafter, the color of the grip area(s) 230 may return to the original color, requesting for two or three further measurements to be performed in the same sequence of steps. Only after receiving data of a certain number (e. g. (exemplarily only, not restricting the invention) three) of measurements of the status and/or color, as far as relevant for determining the applicable actinic irradiation dose, of the user's skin on his/her skin (cutis), the apparatus 100 will start an irradiation of the user's skin with actinic radiation, based - with respect on irradiation dose and irradiation time - directly on the data received from the device 200 in the course of the measurements performed. The advantageous change of the illumination colors appearing at the grip areas 230 thereby shows to the user that the measurement(s) were performed appropriately and the data were transmitted to the control means of the irradiation apparatus 100 controlling the dose and time of the actinic light irradiation "administered" to the user. Thereby, a signal is sent to the user ensuring that an appropriate measurement and data transfer forms the basis for the actinic light irradiation.

With respect to the above features of the device 200 for a measurement of the status and/or color, as far as relevant for determining the applicable actinic irradiation dose, of the user's skin on his/her skin (cutis), in accordance with the present invention, it is specifically mentioned that the device may comprise those features as claimed in claim 1 in combination with one single feature of the above detailed description or may comprise those features as claimed in claim 1 in combination with several or even with all other features of the above detailed description. Particularly in cases where specific advantages are achieved, features useful in achieving these advantages may be combined with all other features described above, particularly with the features claimed in the dependent claims.

The device 200 constructed in accordance with the above description and optionally comprising further means as, for example, microswitches, cables, plugs and/or an unbreakable and humidity-resistant housing may be used for a measurement of the status as far as relevant for determining the actinic irradiation dose, for example the UV radiation dose and/or the visible radiation dose and/or the IR radiation dose, of the skin of a human person, or user, in a number of applications. Such a measurement of the status of the skin is conducted, for example, in connection with an irradiation of the skin with radiation comprising a UV radiation portion in a usual tanning apparatus having artificial UV radiation sources as, for example, UV radiator tubes or high pressure burners. The intensity of those UV radiation sources may, in particularly preferred embodiments of the invention, be controlled in accordance with the status, as far as relevant for determining the UV irradiation dose, of the skin of the user in such a tanning apparatus. In a particularly preferred embodiment, such control may be conducted separately for different parts of the body in accordance with their skin color or their skin tan status, respectively. Thus, darker and/or pre-pigmented parts of the skin, e.g. the skin of the face and/or of the arms, may be irradiated with a stronger intensity and/or for a longer period of time than whiter and/or less (or not at all) pre-pigmented parts of the skin (chest, back). The invention is described in relation to the latter embodiment, without being restricted to this embodiment.

Another area of application for the device 200, which is also relevant in accordance with the invention and is, hence, a preferred area of application, is the measurement of the status, as far as relevant for determining the UV irradiation dose, of the skin of a person in connection with an irradiation of radiation comprising a UV radiation portion in the nature. Specific examples for this embodiment (without the invention being restricted to those embodiments) may be all preparatory steps for a stay on the beach or on the water (sailing, surfing) or for sports activities where a natural sun ray irradiation having a natural UV portion has to be expected.

A further application of the device 200, which is also preferred in accordance with the invention, is a measurement of the status, as far as relevant for determining the UV irradiation dose, of the skin of a person in connection with a medical irradiation of the skin with radiation comprising a UV radiation portion. Certain diseases of the skin as, for example, acne or psoriasis (to name only two of them) may be cured by irradiating UV radiation to the skin struck by said disease or at least, may be moderated considerably with respect to their symptoms. Often, a UV irradiation accompanies the administration of medicaments or may allow the drug dose administered to be reduced or may even replace such drug administration. A controlled irradiation, i.e. an irradiation adjusted, with respect to intensity and duration, to specific parts of the body struck by said disease, may accelerate the curing process, particularly at those parts of the body struck by the disease, without the erythema risk being increased, which risk prevents a continuous healing.

Another area of application for the device 200, which is also preferred in accordance with the invention, is a measurement of the status, as far as relevant for determining the UV irradiation dose, of the skin of a person in connection with a cosmetic irradiation of the skin with radiation comprising a UV radiation portion. A tanning of the skin for purely cosmetic reasons, for improving the appearance, for providing a more healthy and firm skin and for strengthening a good health (optionally also for preparing a sojourn in climates different from at home, where a more intensive exposition of the body to irradiation is expected) gain more and more emphasis. In such a case, a protection against excessive UV radiation becomes more important. The present invention, in a particularly advantageous way, provides good preconditions for avoiding a too strong irradiation and contributes to avoiding erythemas, which are contrary to the aim of an application of UV irradiation in the cosmetic area.

Another area of application for the device 200, which is also preferred in accordance with the invention, is a measurement of the status, as far as relevant for determining the visible light and/or IR light irradiation dose, of the skin of a person in connection with a cosmetic irradiation of the skin with radiation comprising a visible and/or IR radiation portion. An irradiation of the skin for purely cosmetic reasons, for improving the appearance, for providing a more healthy and firm skin and for strengthening a good health, e. g. by improving keratinogenesis in the skin were recently proposed. In such a case, a protection against excessive visible and/or IR radiation was recommended, even if no UV radiation portion was included into the irradiation applied. The present invention, in a particularly advantageous way, provides good preconditions for avoiding a too strong irradiation and contributes to avoiding excessive irradiation with actinic radiation, which are contrary to the aim of an application of actinic radiation, particularly of visible and/or IR irradiation, in the cosmetic area. Reference is made to the above-cited prior art documents, specifically to the European patent application No. 11 002 217.5 (filed 17 March 2011), where a control of actinic radiation irradiation to the human body was proposed, which actinic radiation is, for example, in ranges of wavelengths longer than the UV part of the actinic radiation spectrum, e. g. in the visible wavelengths range of, for example, 450 to 800 nm, and/or in the IR wavelengths range of, for example, > 800 nm, of which the wavelengths ranges of from 570 nm to 610 nm, from 610 nm to 650 nm, from 650 nm to 690 nm and/or from 740 nm to 780 nm and/or from 810 nm to 850 nm are preferred, to address typical, but not restricting examples.

The invention (as mentioned above) also relates to an apparatus for irradiating actinic radiation onto the body of a user. Such apparatus are well known from the prior art and are, particularly, described in the prior patent document EP-A 1 508 301.

Reference is now made to Figure 8. In accordance with the present invention, there is also provided an apparatus 100 for irradiating actinic radiation onto the body of a user. The apparatus 100 comprises at least one means 106 having a surface 102 facing the user and being transparent for the radiation, said apparatus having several groups of UV radiation sources 104 arranged on that side of the surface 102 which is turned away from the user, which UV radiation sources 104 are designed, with respect to their number and position, in such a way, that they can direct radiation comprising a UV radiation portion substantially separately onto different parts of the user's body and which are controllable by one or more means 108 for controlling the power of the UV radiation sources 104, wherein the power control is effected in accordance with the results of the measurements of the status, as far as relevant for determining the UV irradiation dose, of the user's skin at several points of his/her skin (cutis).

In a preferred embodiment of the invention, the apparatus 100 may be a usual tanning apparatus the UV radiation sources 104 of which are designed, in accordance with the aims of the present invention, in such a way that single groups of UV radiation sources 104 may be directed onto different parts of the user's body, and that the respective UV radiation sources 104 directed to different parts of the user's body may be controlled separately with respect to the irradiation intensity and/or radiation duration. As UV radiation sources 104, there may be used, in particularly preferred embodiments of the invention, e.g. UV radiation tubes or high pressure burners or low pressure burners.

In another preferred embodiment of the invention, the apparatus 100 may be an actinic radiation irradiation apparatus the actinic radiation sources 104 of which emit actinic radiation with the exception of UV radiation, e. g. visible light radiation and/or IR radiation, or may emit actinic radiation selected from UV radiation, visible light radiation, IR radiation and radiation selected from two or all three of the above radiation ranges. One example of selected wavelengths radiation, which should, however, not restrict the present invention, is radiation in the visible wavelengths range of, for example, 450 to 800 nm, and/or in the IR wavelengths range of, for example, > 800 nm, or actinic radiation emitted in the wavelengths ranges of from 570 nm to 610 nm, from 610 nm to 650 nm, from 650 nm to 690 nm and/or from 740 nm to 780 nm and/or from 810 nm to 850 nm.

The radiation sources 104 are designed, in accordance with the aims of the present invention, in such a way that single groups of UV radiation sources 104 and/or visible light radiation sources 104 and/or IR radiation sources 104 may be directed onto different parts of the user's body, and that the respective actinic radiation sources 104 directed to different parts of the user's body may be controlled separately with respect to the irradiation intensity and/or radiation duration. As actinic radiation sources 104, there may be used, in particularly preferred embodiments of the invention, e.g. UV radiation tubes and/or high pressure burners and/or low pressure burners and/or LED's and/or other suitable actinic radiation sources known to a skilled person to emit a suitable actinic radiation emission spectrum having any beneficial effect to the apparatus user's body.

In accordance with the invention, a control of the power of the actinic radiation sources 104 is effected in accordance with the results of measurements of the color and/or status, as far as relevant for determining the actinic irradiation dose, of the skin of the person using the apparatus 100, e.g. a tanning apparatus 100 or other actinic radiation irradiating apparatus, before an irradiation and/or simultaneously with the irradiation. In a preferred embodiment of the apparatus 100 according to the invention, a control of the power of the actinic radiation sources 104 is effected in accordance with the results of the measurements of the user's skin color and/or of the user's skin status at the time of interest, preferably before the beginning of the session of irradiating actinic radiation onto the user's body. In an even more preferred embodiment of the invention, such a measurement of the user's skin color and/or of the user's skin status (e. g. the user's skin tan status) is effected by means of one or more devices 200 as they are described above in detail by referring to figures 1 to 7. On the basis of the results of the measurements achieved with the device(s) 200, which determine(s) the reflection of radiation having a defined spectral composition and radiated onto the skin, in relation to the overall radiation irradiated, the devices 108 in the apparatus 100 are addressed, which directly control the power of the actinic radiation sources 104.

In another preferred embodiment of the invention, the number of groups of actinic radiation sources 104 in the apparatus 100 is at least equal to or even larger than the number of points of the user's skin (cutis), where a measurement of the status, as far as relevant for determining the actinic irradiation dose, of the user's skin is effected, more preferred where measurements of the skin color and/or of the skin status (e. g. the skin tan status) of the user's skin are effected. In other words: For a certain apparatus 100, it has to be decided at how many points of the skin (cutis) of the user of the apparatus 100 measurements of, for example, the skin color and/or of the skin status are conducted, i.e. how many different steps of irradiating the user's body should be selected on the basis of the condition of the user's skin. In accordance with such a decision, the number of actinic radiation sources separately controllable with respect to their intensity or radiation duration, respectively, is selected to be equal to or larger than the number of points of measurement in order to achieve an actinic light irradiation of the skin exactly adapted to the status of different parts of the user's body without taking a risk of erythemas or other irritations occurring due to a too strong irradiation of actinic radiation.

To give just a few examples: It is advantageous in accordance with the invention to provide groups of means 108 for controlling the power and/or the irradiation intensity of UV radiation sources 104 in accordance with the results of measurements of the status, as far as relevant for determining the actinic irradiation dose, of the user's skin, in particular measurements of the skin color and/or of the skin status of the user of the apparatus 100, at several points of his/her skin (cutis), which are selected from skin of the face, skin of the chest, skin of the back, skin of the belly, skin of the bottom, skin of the arms and skin of the legs.

In accordance with another preferred embodiment of the invention, the apparatus 100 may be controlled in such a way that the device(s) 200 of the invention take(s) a measurement of the skin color and/or of the skin tan status of the user before starting the irradiation of the actinic light irradiation. In accordance with the result of the measurement, the intensity of the actinic radiation sources 104 and/or the duration of the irradiation are controlled by means of the device(s) 108 in a manner adjusted to the user's skin color and/or to the user's skin status.

In another embodiment of the invention which improves the co-ordination and, hence, is further preferred, the device(s) 200 effect(s) a measurement of the user's skin color and/or of the user's skin status not only before beginning the irradiation, but additionally at least once in the course of the irradiation, further preferred intermittently or continuously in the course of the irradiation. This embodiment has the advantage of allowing to achieve results of measurements also during the irradiation which results come up as a reaction to the irradiation, so that changes of the parameters (skin color, skin status) resulting from an actinic radiation irradiation in the course of the treatment may be taken into account.

In a particularly preferred embodiment, the apparatus 100, which, for example, may be a usual tanning apparatus in cases where the actinic radiation is UV-A and/or UV-B radiation, but also may be an apparatus irradiating radiation selected from UV radiation, visible light radiation, IR radiation and radiation selected from two or all three of the above radiation ranges and may have the features described above in detail, may comprise
(a) a bed 110 with a surface 112 for the user transparent to actinic radiation emitted, said bed having actinic radiation sources 114 emitting actinic radiation;
(b) a movable upper part 120 with a protection surface 122 transparent for the actinic radiation emitted and having actinic radiation sources 124 emitting such actinic radiation;
(c) wherein the upper part 120, in relation to the bed 110, may be opened for allowing a user to get in and may be closed before starting the irradiation step;
(d) control means 130 allowing a user to control the time of the irradiation step; and
(e) one or more means 108 for allowing a separate control of the power of the actinic radiation sources 114, 124, where the control of the power is effected in direct accordance with the results of measurements of the skin color and/or of the skin status of the user at several points of his skin (cutis) by means of the device 200 as described above in detail.

Particularly advantageous for the present invention is the direct control of the power of the actinic radiation sources 114, 124, as a result of the measurement of the user's skin color and/or the user's skin status: There is no intermediate storing, calculation or similar means effecting the control step. Quite to the contrary: The power of the actinic light-emitting sources 114, 124, is effected immediately as a result of the determination of the user's skin color and/or of the user's skin status, and the actinic radiation source's immediate power control means (each of the actinic light emitting sources is controlled by a separate control means) is addressed directly by the means 108 which, in turn, is directly addressed by the device 200 of the invention.

The above-described apparatus 100 is not restricted to the above-referenced embodiment with respect to its design as, for example, a usual tanning apparatus wherein a user is lying on a bed and is irradiated by UV radiation emitted from UV radiation sources 114, 124, which are positioned below the bed's surface 112 and above the protection surface 122. Quite to the contrary, the above-described embodiment does also comprise so-called stand-up irradiation apparatus. In such a stand-up irradiation apparatus, the actinic radiation sources are arranged vertically, and the user is standing and is surrounded by two "halves" of the apparatus 100 wherein the actinic light irradiation sources 114, 124 are arranged around the user in a substantially vertical arrangement.

There is also described a process for irradiating, onto a user's body, actinic radiation from actinic radiation sources emitting such radiation, the process as such not being part of the invention. Said process comprises the step of measuring the status, as far as relevant for determining the actinic irradiation dose, of the user's skin at several points of the skin (cutis), and, in particular, measuring the skin color and/or the skin status of the user's skin, and controlling the power of single groups of said actinic radiation sources, which can direct their actinic radiation substantially separately onto different parts of the user's body, in accordance with the results of said measurements performed with the device 200 of the present invention described above in detail.

Actinic radiation irradiated in accordance with the described process may be any radiation of the actinic wavelength range, as addressed above. Preferably, the actinic radiation may be the UV radiation emitted by usual tanning apparatus light sources. Such tanning apparatus UV light sources operate in such a way that single groups of UV radiation sources are directed onto different parts of the user's body, and that the respective UV radiation sources directed to different parts of the user's body may be controlled separately with respect to the irradiation intensity and/or radiation duration. As UV radiation sources 104, there may be used, in particularly preferred embodiments of the process of the invention, e.g. UV radiation tubes or high pressure burners or low pressure burners or LED's.

In another example, the described process may comprise irradiating actinic radiation with the exception of UV radiation, e. g. visible light radiation and/or IR radiation, or may comprise irradiating actinic radiation selected from UV radiation, visible light radiation, IR radiation and radiation selected from two or all three of the above radiation ranges. One example of selected wavelengths radiation employed in the process of the invention, which should, however, not restrict the present invention, is radiation in the visible wavelengths range of, for example, 450 to 800 nm, and/or in the IR wavelengths range of, for example, > 800 nm, or actinic radiation emitted in the wavelengths ranges of from 570 nm to 610 nm, from 610 nm to 650 nm, from 650 nm to 690 nm and/or from 740 nm to 780 nm and/or from 810 nm to 850 nm.

In a specific case of the measurement of the skin status, as far as relevant for a UV irradiation, the device 200 is activated, and a measurement of the skin status of the user is effected at at least two points of the user's body subsequently, generally at n points of the user's body. Those points may be selected, for example, from skin of the face, skin of the chest, skin of the back, skin of the belly, skin of the bottom, skin of the arms and skin of the legs without restricting the invention to such measurements.

The measurement itself is effected, as partly already described above, in the following manner by using the device 200 described above as particularly useful for such a measurement:

The flat and rigid surface area 207 (e. g. the measurement window 207) at the "bottom" of the device 200 is brought in contact to the user's skin. When the user does so, the whole unit may move to an extent of a certain angle (relative to the axis A of the device 200) due to the fact that the membrane 208 is flexible, in order to seek the ideal measurement position. In the course of such movements, the whole unit (including the rigid part 206, the measurement window 207, the Ulbricht sphere 205, the LED's 201, the focusing lens 202 a and the photodiode/sensor 202 as well as the Hall sensor 209 as the actuating means 209) may follow the same movement, when seeking the ideal measurement position. Such an ideal measurement position is found as soon as the measurement window 207 (i. e. the flat and rigid surface area 207) has the correct position relative to the user's skin, e. g. a position completely parallel to the "lower") measurement window 207.

The magnet(s) 210, preferably the one magnet 210, positioned "above" the Hall sensor 209 in the housing 200 a of the device 200 are/is arranged in the housing 200 a of the device 200 in such a position that its (or their) magnetic field(s) extend(s) into such an area that, at the ideal measurement position of the unit containing all the other parts described above and contributing to the measurement, the Hall sensor(s) 209 has/have been moved into the area of the existing magnetic field generated by the magnet(s) 210. This may, for example and without restriction, be the area immediately above the middle (e. g. in the axial position) above the Hall sensor 209. As a consequence of the Hall sensor's 209 having approached the magnet(s) 210 closer than the threshold distance of the magnetic field, the Hall sensor(s) 209 is/are "switched" from "OFF" to "ON" and start(s) the measurement. This is effected by initiating the radiation emission means 201 (e. g. the LED's 201) to emit radiation towards the diffusing means 205 (e. g. the Ulbricht sphere 205), where the radiation is made diffuse. The radiation is proceeding towards the walls of the Ulbricht sphere 205 and, inter alia, to the area where the flat and rigid surface area 207 is positioned in a fixed manner. The radiation passes the measurement window 207, impinges onto the user's skin, is (in part) reflected (in part) back by the user's skin through the measurement window 207 and proceeds towards the focusing means 202 a (e. g. the lens 202 a) and to the sensor 202 (e. g. the photodiode 202) where its amount and wavelength range is measured.

The sensor 202 sends the signal immediately to the means controlling the power and intensity of the actinic radiation sources, thereby controlling which, and how much, actinic radiation of a certain wavelength may be irradiated onto the user's skin, and also for what time or irradiation.

The amount of radiation reflected by the user's skin is focused onto the focus of the sensor/photodiode 202 by the focusing means/lens 202 a and measured by the sensor 202. The data obtained are transmitted to the data analyzing unit 203. In said unit, the values of the measurements of the amount of radiation at the measurement point 1, at the measurement point 2, ..., and at the measurement point n may be compared to standard values from which can be concluded how high the maximum radiation dose (or the maximum irradiation time at a certain dose) may be at the point where the first, second, ..., n-th measurement was effected. The actinic radiation irradiation dose (e. g. the UV radiateon irradiation dose) for the first, second, ..., n-th point the user's body is adjusted as a direct adjustment following the data transmitted from the sensor 202/photodiode, wherein the adjustment may be an adjustment of the intensity at a constant time of the whole irradiation, an adjustment of the time at a constant irradiation dose or any combination of parameters providing a guarantee that the skin is irradiated with such an intensity only that the erythema (or other irritation) threshold is not reached.

The actinic radiation source, for example the UV radiation tube or the highpressure burner, and/or the visible radiation source and/or the IR radiation source for the point 1, 2, ..., n is initiated and is switched off again after reaching the maximum value of the dose, of the time and/or of any other parameter desired.

Similarly, measurements are effected in cases of other wavelength ranges of the actinic light spectrum, e. g. selected from UV radiation, visible light radiation, IR radiation and radiation selected from two or all three of the above radiation ranges.

In a particularly preferred embodiment of the invention, the emitted power and/or the emitted actinic radiation since the initiation of the radiation emission may also be measured in the course of the irradiation at the points 1, 2, ..., n of the user's body and may then be compared with the values indicated above to be maximum values. If such a comparison shows that the maximum values for a specific point 1, 2, ..., n, are reached or even exceeded, the actinic light irradiation apparatus will switch off the actinic light radiation sources for an irradiation of that point or those parts of the body singly (or may switch off the whole apparatus).

The number of points of the user's skin (cutis) where a measurement of the relevant parameters as, for example and preferably, the skin color and the skin tan status of the user, may be effected is basically not restricted. It is, however, preferred that the skin color and/or the skin status of the user be measured at at least two points of his/her skin (cutis), and the power of two or more groups of the actinic light radiation sources is controlled in accordance with the results of such measurements. It is particularly preferred in the described process, the process as such not being part of the invention, that the skin color and/or the skin status of the user be measured at least at two points, generally at n points of his/her skin (cutis), which are selected from skin of the face, skin of the chest, skin of the back, skin of the belly, skin of the bottom, skin of the arms and skin of the legs.

In accordance with the described process, the process as such not being part of the invention, it is particularly preferred that the measurement of the skin color and/or of the skin status of the user be effected by means of one or more means 200 measuring the absorption and/or reflection of radiation having a defined spectral composition irradiated onto the skin in relation to the irradiated radiation and that the means 108 for controlling the power of the actinic radiation sources 104 are addressed in accordance with the results of such a measurement.

As already pointed out above in connection with the apparatus 100, the measurement of the status, as far as relevant for determining the actinic radiation dose, of the skin of a user is preferably effected before starting the irradiation. In accordance with the results of such measurements, the irradiation is controlled. In another example a measurement of the parameters, preferably a measurement of the skin color and/or of the skin status of the user, is effected before starting the irradiation and, additionally, at least once in the course of the irradiation, even more preferred intermittently or continuously in the course of the irradiation with actinic radiation. This example is particularly advantageous, since, in the course of the irradiation of actinic radiation, it may be prevented, in emergency cases, by switching off the corresponding radiation sources or even the whole apparatus, that the maximum parameters of the irradiation (time, intensity, etc. possibly) resulting into the generation of erythemas or other irritations of the skin are exceeded, if a measurement of the parameters relevant for the skin shows that the skin at the measurement point is not suitable for such a long and/or intensive irradiation by actinic radiation.

The device of the invention is applied in numerous fields which are not restricted in accordance with the invention. Preferred application fields are processes for a cosmetic skin irradiation and for a medical skin irradiation.

By a measurement of the status, as far as relevant for determining the actinic radiation dose, of the user's skin, preferably by a measurement of the user's skin color and/or the user's skin status, at least before starting the irradiation, it is possible to adjust an irradiation of actinic radiation exactly to the individual parameters of parts of the user's skin having a different sensitivity to UV radiation. An over-exposure resulting into the generation of an erythema ("sunburn") or other irritations of the skin are reliably prevented even for sensitive light or unpigmented skin. This is achieved by a measurement process using the device 200 of the present invention which is simple, quickly to effect and does not need extensive apparatus.

## Claims

1. A device (200) for a measurement of the status and/or color, for determining an applicable actinic irradiation dose, of the skin of a human on his/her skin (cutis), comprising, housed in a rigid housing (200 a),
(a) at least one means (201) for emitting radiation having a defined spectral composition onto the skin (cutis);
(b) at least one sensor (202) for measuring the portion of the radiation irradiated by the radiation-emission means (201) which is reflected by the skin (cutis); and
(c) at least means (212) for providing energy to the at least one emission means (201) and/or to the at least one sensor (202) and for transmitting measured data to a data analyzing means (203);
said device further comprising
(d) in said housing (200 a): at least one means (205) for diffusing the radiation, emitted by the radiation emission means (201), between the radiation emission means (201) and the human's skin (cutis);
(e) in said housing (200 a): at least one means (202 a) for focusing, towards the at least one sensor (202), the portion of the radiation irradiated by the radiation-emission means (201) which is reflected by the skin (cutis);
(f) in said housing (200 a): at least one means (206) capable of fixing the position of said at least one radiation emission means (201), said at least one focusing means (202 a), said at least one sensor (202), and said at least one radiation diffusion means (205) relative to each other; and
(g) at least one flat and rigid surface area (207) capable of allowing the passage of the radiation emitted from said at least one radiation emission means (201) out of said housing (200 a) towards the human's skin (cutis) and of the radiation reflected from the human's skin (cutis) into said housing (200 a) towards the at least one focusing means (202 a) and the at least one sensor (202), **characterized in that** said at least one flat and rigid surface area (207) is included into the arrangement of the four components of (f) above fixed by said fixing means (206); said device further comprising
(h) a flexible membrane (208) which incorporates said flat and rigid surface area (207), and
(i) at least one means (209) disposed within the housing (200 a) and capable of actuating the human's skin status and/or skin color measurement;
or
a device (200) for a measurement of the status and/or color, for determining an applicable actinic irradiation dose, of the skin of a human on his/her skin (cutis), said device (200) consisting of a rigid housing (200 a), and
(a) housed in the rigid housing (200a), one or more means (201) for emitting radiation having a defined spectral composition onto the skin (cutis);
(b) housed in the rigid housing (200a), one or more sensors (202) for measuring the portion of the radiation irradiated by the radiation-emission means (201) which is reflected by the skin (cutis);
(c) housed in the rigid housing (200a), means (212) for providing energy to the one or more emission means (201) and/or to the one or more sensors (202) and for transmitting measured data to a data analyzing means (203);
(d) in said housing (200 a): one or more means (205) for diffusing the radiation, emitted by the radiation emission means (201), between the radiation emission means (201) and the human's skin (cutis);
(e) in said housing (200 a): one or more means (202 a) for focusing, towards the one or more sensors (202), the portion of the radiation irradiated by the radiation-emission means (201) which is reflected by the skin (cutis);
(f) in said housing (200 a): one or more means (206) capable of fixing the position of said one or more radiation emission means (201), said one or more focusing means (202 a), said one or more sensors (202), and said one or more radiation diffusion means (205) relative to each other;
(g) one or more flat and rigid surface areas (207) capable of allowing the passage of the radiation emitted from said one or more radiation emission means (201) out of said housing (200 a) towards the human's skin (cutis) and of the radiation reflected from the human's skin (cutis) into said housing (200 a) towards the one or more focusing means (202 a) and the one or more sensors (202), and means (h) and (i), **characterized in that** said one or more flat and rigid surface areas (207) is included into the arrangement of the four rigid surface area (207) being is included into the arrangement of the four components of (f) above fixed by said fixing means (206); and **in that**
(h) a flexible membrane (208) incorporates said flat and rigid surface area (207), and
(i) one or more means (209) is/are disposed within the housing (200 a) and is/are capable of actuating the human's skin status and/or skin color measurement.

2. The device (200) according to claim 1, wherein said device (200) comprises said flexible membrane (208) in a releasable connection to said housing (200 a).

3. The device (200) according to claim 2, wherein said releasable connection of the flexible membrane (208) to the housing (200 a) is sealed to be impermeable for gases, vapours, liquids and solids.

4. The device (200) according to any of the claims 1 to 3, wherein said at least one actuating means (209) is disposed and fixed on a side of the radiation diffusing means (205) which is opposite to the side of the flat and rigid surface area (207), preferably wherein said at least one actuating means (209) is disposed on a side of the radiation diffusing means (205) which is opposite to the flat and rigid surface area (207) and is disposed and fixed centrally on the central longitudinal axis (A) of the housing (200 a).

5. The device (200) of claim 4, in the case where the device comprises further elements, the device further comprising at least one magnet (210) capable of effecting a contact for actuating said actuating means (209), preferably comprising magnet(s) (210) in combination with a Hall sensor as the actuating means (209).

6. The device (200) according to any of the claims 1 to 5, in the case where the device comprises further elements, the device further comprising the rigid housing having the shape of a truncated cone, preferably having colored grip areas (230), more preferably having grip areas (230) of a light-transparent material, even more preferably having grip areas (230) of a material capable of allowing colored illumination light to pass from the inside towards the outer side, utmost preferably having grip areas (230) of a material capable of allowing colored illumination light to pass from the inside, when addressed by electronic signals relating to measuring functions, towards the outer side, thereby signaling measuring functions to the user.

7. An apparatus (100) for irradiating actinic radiation onto the body of a user, said apparatus comprising at least one means (106) having a surface (102) facing the user and being transparent for the actinic radiation and having several groups of actinic radiation sources (104) arranged on that side of the surface (102) which is turned away from the user, which actinic radiation sources (104) are designed, with respect to their number and position, in such a way that they can direct actinic radiation substantially separately onto different parts of the user's body, and are controllable by one or more means (108) for controlling the power of the actininc radiation sources (104), wherein the power control is effected in accordance with the results of the measurements of the status, for determining an applicable actinic irradiation dose, of the user's skin at several points of his/her cutis, by means of at least one device according to any one or more of the claims 1 to 6.

8. The apparatus (100) according to claim 7, wherein the actinic radiation is actinic radiation comprising at least in part radiation in the UV wavelengths range having a physiological effect, preferably a tanning effect, on the user's skin (cutis); and/or wherein the actinic radiation is actinic radiation comprising at least in part radiation in the visible wavelengths range having a physiological effect on the user's skin (cutis); and/or wherein the actinic radiation is actinic radiation comprising at least in part radiation in the IR wavelengths range having a physiological effect on the user's skin; and/or wherein the actinic radiation is actinic radiation comprising at least in part radiation in at least two of the wavelengths ranges selected from the UV wavelengths range having a physiological effect on the user's skin (cutis), visible wavelengths range having a physiological effect on the user's skin (cutis), and IR wavelengths range having a physiological effect on the user's skin (cutis).

9. The apparatus (100) according to any one or more of the claims 7 or 8, wherein at least one of the means (200) is/are capable of taking a measurement of the user's skin color and/or of the user's skin status before beginning the irradiation.

10. The apparatus (100) according to claim 9, wherein the at least one means (200) is/are capable of taking additional measurements of the user's skin color and/or of the user's skin tan status - optionally additionally - at least once in the course of the irradiation, preferably intermittently or continuously in the course of the irradiation.

11. The apparatus (100) according to any one or more of the claims 7 to 10, comprising several means (108) for controlling the power of the UV radiation sources (104) in accordance with, and upon direct influence by, the results of the measurements of the user's skin type and/or the user's skin status at several points of the user's skin (cutis), preferably selected from the user's skin of the face, the user's skin of the chest, the user's skin of the back, the user's skin of the belly, the user's skin of the bottom, the user's skin of the arms and the user's skin of the legs.

12. The apparatus (100) according to any one or more of the claims 7 to 11, wherein the measurement of the user's skin color and/or of the user's skin status is effected by means of one or more means (200) according to any one or more of the claims 1 to 6, and the means (108) controlling the power of the actinic radiation sources (104) are addressed directly in accordance with results of said measurement of the user's skin color and/or of the user's skin status.

13. The apparatus (100) according to any of the claims 7 to 12, comprising
(a) a bed (110) with a surface (112) for the user transparent for the actinic radiation, and actinic radiation sources (114) emitting actinic radiation;
(b) a movable upper part (120) having a protection surface (122) transparent for the actinic radiation, and actinic radiation sources (124) emitting actinic radiation;
(c) wherein the upper part (120), in relation to the bed (110) may be opened for allowing a user to get in and may be closed before starting the irradiation process;
(d) controlling means (130) for controlling the time of an irradiation process which may be used by the user; and
(e) one or more device(s) (108) for separately controlling the power of the actinic radiation sources (114, 124), where the control of the power is effected in accordance with the results of measurements of the user's skin color and/or of the user's skin status at several points of his/her skin (cutis) by means of the device (200) as claimed in any of the claims 1 to 6.

14. A device (200) of any of the claims 1 to 6 for use in a process for irradiating, to a user's body, actinic radiation from actinic radiation sources emitting such radiation, said device (200) being capable of enabling the steps of measuring the status and/or the color, for determining an applicable actinic irradiation dose, of the user's skin at several points of the skin (cutis), and of controlling the power of single groups of said actinic radiation sources capable of directing their radiation substantially separately onto different parts of the user's body, in accordance with the results of said measurements effected by means of said device (200).

15. The device (200) according to claim 14 for use in a process for irradiating, to a user's body, actinic radiation from actinic radiation sources emitting such radiation, said device (200) being capable of enabling the steps of measuring the user's skin color or skin status at at least two points of the skin (cutis) and of controlling the power of two or more than two groups of actinic radiation sources in accordance with the results of said measuring steps, preferably being capable of enabling the steps of measuring the user's skin color or skin status at at least two points of the skin (cutis), which are selected from skin of the face, skin of the chest, skin of the back, skin of the belly, skin of the bottom, skin of the arms and skin of the legs.

16. The device (200) according to claim 14 or claim 15 for use in a process for irradiating, to a user's body, actinic radiation from actinic radiation sources emitting such radiation, said device (200) being capable of enabling the step of measuring the user's skin color/type and/or the user's skin status before initiating the irradiation, or said device (200) being capable of enabling the step of measuring the user's skin color/type and/or the user's skin status additionally at least once in the course of the irradiation, preferably intermittently or continuously in the course of the irradiation, with actinic radiation.

17. The device (200) according to any of the claims 14 to 16 for use in a process of cosmetic skin irradiation or for use in a process of medical skin irradiation.

## Patentansprüche

1. Vorrichtung (200) für eine Messung des Status und/oder der Farbe, zum Bestimmen einer anwendbaren Dosis aktinischer Strahlung, der Haut eines Menschen auf seiner/ihrer Haut (cutis), umfassend - untergebracht in einem starren Gehäuse (200 a) -
(a) wenigstens eine Einrichtung (201) zum Emittieren von Strahlung, die eine definierte spektrale Zusammensetzung aufweist, auf die Haut (cutis);
(b) wenigstens einen Sensor (202) zum Messen des Teils der durch die Strahlungs-Emissions-Einrichtung (201) eingestrahlten Strahlung, die durch die Haut (cutis) reflektiert wird; und
(c) wenigstens eine Einrichtung (212) zum Liefern von Energie zu der wenigstens einen Emissions-Einrichtung (201) und/oder zu dem wenigstens einen Sensor (202) und zum Übertragen gemessener Daten an eine Daten-Analyse-Einrichtung (203);
wobei die Vorrichtung weiter umfasst
(d) in dem Gehäuse (200 a): wenigstens eine Einrichtung (205) zum Zerstreuen der Strahlung, die von der Strahlungs-Emissions-Einrichtung (201) emittiert wird, zwischen der Strahlungs-Emissions-Einrichtung (201) und der Haut (cutis) des Menschen;
(e) in dem Gehäuse (200 a): wenigstens eine Einrichtung (202 a) zum Fokussieren des Teils der von der Strahlungs-Emissions-Einrichtung (201) eingestrahlten Strahlung, der von der Haut (cutis) reflektiert wird, auf den wenigstens einen Sensor (202);
(f) in dem Gehäuse (200 a): wenigstens eine Einrichtung (206), die befähigt ist zum Fixieren der Position der wenigstens einen Strahlungs-Emissions-Einrichtung (201), der wenigstens einen Fokussierungs-Einrichtung (202 a), des wenigstens einen Sensors (202) und der wenigstens einen Strahlung zersteuenden Einrichtung (205) relativ zueinander; und
(g) wenigstens einen flachen und starren Oberflächenbereich (207), der befähigt ist, den Durchtritt der Strahlung, die von der wenigstens einen Strahlungs-Emissions-Einrichtung (201) aus dem Gehäuse (200 a) in Richtung auf die Haut (cutis) des Menschen emittiert wird, und der Strahlung, die von der Haut (cutis) des Menschen in das Gehäuse (200 a) und in Richtung auf die wenigstens eine Fokussierungs-Einrichtung (202 a) und den wenigstens einen Sensor (202) reflektiert wird, zu erlauben;
**dadurch gekennzeichnet, dass** der wenigstens eine flache und starre Oberflächen-Bereich (207) eingeschlossen ist in die Anordnung der durch die Fixier-Einrichtung (206) fixierten vier Komponenten aus dem obigen Absatz (f), wobei die Vorrichtung weiter umfasst
(h) eine flexible Membran (208), die den flachen und starren Oberflächen-Bereich (207) inkorporiert; und
(i) wenigstens eine Einrichtung (209), die in dem Gehäuse (200 a) angeordnet ist und befähigt ist, die Messung des Status der Haut und/oder der Farbe der Haut des Menschen auszulösen;
oder
Vorrichtung (200) für eine Messung des Status und/oder der Farbe, zum Bestimmen einer anwendbaren Dosis aktinischer Strahlung, der Haut eines Menschen auf seiner/ihrer Haut (cutis), bestehend aus einem starren Gehäuse (200 a), und
(a) in dem starren Gehäuse (200 a) untergebracht: einer oder mehreren Einrichtung(en) (201) zum Emittieren von Strahlung, die eine definierte spektrale Zusammensetzung aufweist, auf die Haut (cutis);
(b) in dem starren Gehäuse (200 a) untergebracht: einem oder mehreren Sensor(en) (202) zum Messen des Teils der durch die Strahlungs-Emissions-Einrichtung (201) eingestrahlten Strahlung, die durch die Haut (cutis) reflektiert wird;
(c) in dem starren Gehäuse (200 a) untergebracht: einer Einrichtung (212) zum Liefern von Energie zu der einen oder mehreren Emissions-Einrichtung(en) (201) und/oder zu dem einen oder mehreren Sensor(en) (202) und zum Übertragen gemessener Daten an eine Daten-Analyse-Einrichtung (203);
(d) in dem Gehäuse (200 a): einer oder mehreren Einrichtung(en) (205) zum Zerstreuen der Strahlung, die von der Strahlungs-Emissions-Einrichtung (201) emittiert wird, zwischen der Strahlungs-Emissions-Einrichtung (201) und der Haut (cutis) des Menschen;
(e) in dem Gehäuse (200 a): einer oder mehreren Einrichtung(en) (202 a) zum Fokussieren des Teils der von der Strahlungs-Emissions-Einrichtung (201) eingestrahlten Strahlung, der von der Haut (cutis) reflektiert wird, auf den einen oder mehrere Sensor(en) (202);
(f) in dem Gehäuse (200 a): einer oder mehreren Einrichtung(en) (206), die befähigt ist/sind zum Fixieren der Position der einen oder mehreren Strahlungs-Emissions-Einrichtung(en) (201), der einen oder mehreren Fokussierungs-Einrichtung(en) (202 a), des einen Sensors oder der mehreren Sensoren (202) und der einen oder der mehreren Strahlung zersteuenden Einrichtung(en) (205) relativ zueinander;
(g) einem oder mehreren flachen und starren Oberflächenbereich(en) (207), der/die befähigt ist/sind, den Durchtritt der Strahlung, die von der einen oder den mehreren Strahlungs-Emissions-Einrichtung(en) (201) aus dem Gehäuse (200 a) in Richtung auf die Haut (cutis) des Menschen emittiert wird, und der Strahlung, die von der Haut (cutis) des Menschen in das Gehäuse (200 a) in Richtung auf die eine oder mehreren Fokussierungs-Einrichtung(en) (202 a) und den einen Sensor oder die mehreren Sensoren (202) reflektiert wird, zu erlauben; und Einrichtungen (h) und (i),
**dadurch gekennzeichnet, dass** der eine oder die mehreren flache(n) und starre(n) Oberflächen-Bereich(e) (207) eingeschlossen ist/sind in die Anordnung der durch die Fixier-Einrichtung (206) fixierten vier Komponenten aus dem obigen Absatz (f), und dadurch, dass
(h) eine flexible Membran (208) den flachen und starren Oberflächen-Bereich (207) inkorporiert; und
(i) eine oder mehrere Einrichtung(en) (209) in dem Gehäuse (200 a) angeordnet ist/sind und befähigt ist/sind, die Messung des Status der Haut und/oder der Farbe der Haut des Menschen auszulösen.

2. Vorrichtung (200) nach Anspruch 1, wobei die Vorrichtung (200) die flexible Membran (208) in einer lösbaren Verbindung zu dem Gehäuse (200 a) umfasst.

3. Vorrichtung (200) nach Anspruch 2, worin die lösbare Verbindung der flexiblen Membran (208) zu dem Gehäuse (200 a) so abgedichtet ist, dass sie undurchlässig ist für Gase, Dämpfe, Flüssigkeiten und Feststoffe.

4. Vorrichtung (200) nach irgendeinem der Ansprüche 1 bis 3, wobei die wenigstens eine Auslöse-Einrichtung (209) auf einer Seite der die Strahlung zerstreuenden Einrichtung (205) angeordnet und fixiert ist, die gegenüber der Seite des flachen und starren Oberflächen-Bereichs (207) liegt, vorzugsweise wobei die wenigstens eine Auslöse-Einrichtung (209) auf einer Seite der die Strahlung zerstreuenden Einrichtung (205) angeordnet ist, die gegenüber der Seite des flachen und starren Oberflächen-Bereichs (207) liegt, und zentral auf der zentralen Längsachse (A) des Gehäuses (200 a) angeordnet und fixiert ist.

5. Vorrichtung (200) nach Anspruch 4, in dem Fall, in dem die Vorrichtung weitere Elemente umfasst, wobei die Vorrichtung weiter umfasst: wenigstens einen Magneten (210), der befähigt ist, einen Kontakt zum Auslösen der Auslöse-Einrichtung (209) herzustellen, vorzugsweise umfassend (einen) Magneten (210) in Kombination mit einem Hall-Sensor als Auslöse-Einrichtungen (209).

6. Vorrichtung (200) nach irgendeinem der Ansprüche 1 bis 5, in dem Fall, in dem die Vorrichtung weitere Elemente umfasst, wobei die Vorrichtung weiter umfasst: das starre Gehäuse mit der Form eines Kegelstumpfs, vorzugsweise gefärbte Greif-Bereiche (230) aufweisend, weiter bevorzugt Greif-Bereiche (230) aus einem für Licht transparenten Material aufweisend, noch weiter bevorzugt Greif-Bereiche (230) aus einem Material aufweisend, das befähigt ist, zu erlauben, dass farbiges Illuminations-Licht von der Innenseite in Richtung auf die Außenseite hindurchtritt, am meisten bevorzugt Greif-Bereiche (230) aus einem Material aufweisend, das befähigt ist, zu erlauben, dass farbiges Illuminations-Licht von der Innenseite in Richtung auf die Außenseite hindurchtritt, wenn diese mittels elektronischer Signale angesteuert werden, die mit messenden Funktionen in Beziehung stehen, wodurch dem Benutzer Mess-Funktionen signalisiert werden.

7. Apparat (100) zum Einstrahlen aktinischer Strahlung auf den Körper eines Benutzers, wobei der Apparat wenigstens eine Einrichtung (106) umfasst, die eine zu dem Benutzer hin zeigende und für die aktinische Strahlung transparente Oberfläche (102) aufweist und einige Gruppen von Quellen (104) für aktinische Strahlung aufweist, die auf der Seite der Oberfläche (102) angeordnet sind, die von dem Benutzer weg zeigt, wobei die Quellen (104) für aktinische Strahlung in Bezug auf ihre Zahl und Position in der Weise ausgelegt sind, dass sie aktinische Strahlung im Wesentlichen getrennt auf verschiedene Teile des Körpers des Benutzers richten können, und durch eine oder mehrere Einrichtung(en) (108) zum Steuern der Energie der Quellen (104) für aktinische Strahlung steuerbar sind, wobei die Energie-Steuerung bewirkt wird in Übereinstimmung mit den Ergebnissen der Messungen des Status, zum Bestimmen einer anwendbaren Dosis aktinischer Strahlung, der Haut des Benutzers an einigen Punkten seiner/ihrer cutis, mittels wenigstens einer Vorrichtung gemäß irgendeinem oder mehreren der Ansprüche 1 bis 6.

8. Apparat (100) nach Anspruch 7, wobei die aktinische Strahlung aktinische Strahlung ist, die wenigstens zum Teil Strahlung im UV-Wellenlängen-Bereich umfasst, die eine physiologische Wirkung, vorzugsweise eine Bräunungs-Wirkung, auf der Haut (cutis) des Benutzers aufweist; und/oder worin die aktinische Strahlung aktinische Strahlung ist, die wenigstens zum Teil Strahlung im sichtbaren Wellenlängen-Bereich umfasst, die eine physiologische Wirkung auf der Haut (cutis) des Benutzers aufweist; und/oder worin die aktinische Strahlung aktinische Strahlung ist, die wenigstens zum Teil Strahlung im IR-Wellenlängen-Bereich umfasst, die eine physiologische Wirkung auf der Haut des Benutzers aufweist; und/oder worin die aktinische Strahlung aktinische Strahlung ist, die wenigstens zum Teil Strahlung in wenigstens zwei der Wellenlängen-Bereiche aufweist, die gewählt sind aus dem UV-Wellenlängen-Bereich, der eine physiologische Wirkung auf der Haut (cutis) des Benutzers aufweist, dem sichtbaren Wellenlängen-Bereich, der eine physiologische Wirkung auf der Haut (cutis) des Benutzers aufweist, und dem IR-Wellenlängen-Bereich, der eine physiologische Wirkung auf der Haut (cutis) des Benutzers aufweist.

9. Apparat (100) nach irgendeinem oder mehreren der Ansprüche 7 oder 8, wobei wenigstens eine der Einrichtungen (200) befähigt ist/sind, eine Messung der Farbe der Haut des Benutzers und/oder des Status der Haut des Benutzers vor dem Beginnen der Bestrahlung durchzuführen.

10. Apparat (100) nach Anspruch 9, wobei die wenigstens eine Einrichtung (200) befähigt ist/sind, zusätzliche Messungen der Farbe der Haut des Benutzers und/oder des Status der Haut des Benutzers - gegebenenfalls zusätzlich - wenigstens einmal im Verlauf der Bestrahlung durchzuführen, vorzugsweise intermittierend oder kontinuierlich im Verlauf der Bestrahlung.

11. Apparat (100) nach irgendeinem oder mehreren der Ansprüche 7 bis 10, umfassend einige Einrichtungen (108) zum Steuern der Energie der UV-Strahlungsquellen (104) in Übereinstimmung mit, und auf direkte Beeinflussung durch, die Ergebnisse der Messungen des Typs der Haut des Benutzers und/oder des Status der Haut des Benutzers an einigen Punkten der Haut (cutis) des Benutzers, vorzugsweise gewählt aus der Haut des Gesichts des Benutzers, der Haut der Brust des Benutzers, der Haut des Rückens des Benutzers, der Haut des Bauchs des Benutzers, der Haut des Unterleibs des Benutzers, der Haut der Arme des Benutzers und der Haut der Beine des Benutzers.

12. Apparat (100) nach irgendeinem oder mehreren der Ansprüche 7 bis 11, wobei die Messung der Farbe der Haut des Benutzers und/oder des Status der Haut des Benutzers bewirkt wird mittels einer oder mehreren Einrichtung(en) (200) nach irgendeinem oder mehreren der Ansprüche 1 bis 6, und die Einrichtungen (108), die die Energie der Quellen (104) aktinischer Strahlung steuern, direkt in Übereinstimmung mit den Ergebnissen der Messung der Farbe der Haut des Benutzers und/oder des Status der Haut des Benutzers angesteuert werden.

13. Apparat (100) nach irgendeinem der Ansprüche 7 bis 12, umfassend
(a) ein Bett (110) mit einer Oberfläche (112) für den Benutzer, die transparent für die aktinische Strahlung ist, und Quellen (114) für aktinische Strahlung, die aktinische Strahlung emittieren;
(b) einen bewegbaren oberen Teil (120), der eine Schutzfläche (122) aufweist, die transparent für die aktinische Strahlung ist, und Quellen (124) für aktinische Strahlung, die aktinische Strahlung emittieren;
(c) wobei der obere Teil (120) in Beziehung zu dem Bett (110) geöffnet werden kann, um zu erlauben, dass ein Benutzer einsteigen kann, und geschlossen werden kann, bevor der Bestrahlungs-Vorgang gestartet wird;
(d) Steuer-Einrichtungen (130) zum Steuern der Zeit eines Bestrahlungs-Vorgangs, die von dem Benutzer benutzt werden können; und
(e) eine oder mehrere Vorrichtung(en) (108) zum getrennten Steuern der Energie der Quellen (114, 124) für aktinische Strahlung, wobei die Steuerung der Energie bewirkt wird in Übereinstimmung mit den Ergebnissen der Messungen der Farbe der Haut des Benutzers und/oder des Status der Haut des Benutzers an einigen Punkten seiner/ihrer Haut (cutis) mittels der Vorrichtung (200) gemäß irgendeinem der Ansprüche 1 bis 6.

14. Vorrichtung (200) nach irgendeinem der Ansprüche 1 bis 6 zur Verwendung in einem Verfahren zum Einstrahlen aktinischer Strahlung auf den Körper eines Benutzers von Quellen aktinischer Strahlung, die derartige Strahlung emittieren, wobei die Vorrichtung (200) befähigt ist zum Ermöglichen der Schritte des Messens des Status und/oder der Farbe, zum Bestimmen einer anwendbaren Dosis aktinischer Strahlung, der Haut des Benutzers an einigen Punkten der Haut (cutis) und des Steuerns der Energie einzelner Gruppen der Quellen aktinischer Strahlung, die befähigt sind, ihre Strahlung im Wesentlichen getrennt auf verschiedene Teile des Körpers des Benutzers zu richten, in Übereinstimmung mit der Ergebnissen der Messungen, die durch die Vorrichtung (200) bewirkt werden.

15. Vorrichtung (200) nach Anspruch 14 zur Verwendung in einem Verfahren zum Einstrahlen aktinischer Strahlung auf den Körper eines Benutzers von Quellen aktinischer Strahlung, die derartige Strahlung emittieren, wobei die Vorrichtung (200) befähigt ist zum Ermöglichen der Schritte des Messens der Farbe der Haut des Benutzers oder des Status der Haut des Benutzers an wenigstens zwei Punkten der Haut (cutis) und des Steuerns der Energie von zwei oder mehr als zwei Gruppen der Quellen aktinischer Strahlung in Übereinstimmung mit den Ergebnissen der Mess-Schritte, vorzugsweise wobei die Vorrichtung (200) befähigt ist zum Ermöglichen der Schritte des Messens der Farbe der Haut des Benutzers oder des Status der Haut des Benutzers an wenigstens zwei Punkten der Haut (cutis), die gewählt sind aus der Gruppe Haut des Gesichts, Haut der Brust, Haut des Rückens, Haut des Bauchs, Haut des Unterleibs, Haut der Arme und Haut der Beine.

16. Vorrichtung (200) nach Anspruch 14 oder Anspruch 15 zur Verwendung in einem Verfahren zum Einstrahlen aktinischer Strahlung auf den Körper eines Benutzers von Quellen aktinischer Strahlung, die derartige Strahlung emittieren, wobei die Vorrichtung (200) befähigt ist zum Ermöglichen der Schritte des Messens der Farbe/des Typs der Haut des Benutzers und/oder des Status der Haut des Benutzers vor dem Beginnen der Bestrahlung, oder wobei die Vorrichtung (200) befähigt ist zum Ermöglichen der Schritte des Messens der Farbe/des Typs der Haut des Benutzers und/oder des Status der Haut des Benutzers zusätzlich wenigstens einmal im Verlauf der Bestrahlung, vorzugsweise intermittierend oder kontinuierlich im Verlauf der Bestrahlung, mit aktinischer Strahlung.

17. Vorrichtung (200) nach irgendeinem der Ansprüche 14 bis 16 zur Verwendung in einem Verfahren einer kosmetischen Haut-Bestrahlung oder zur Verwendung in einem Verfahren zur medizinischen Haut-Bestrahlung.

## Revendications

1. Dispositif (200) pour une mesure de l'état et/ou de la couleur, pour déterminer une dose de rayonnement actinique applicable, de la peau d'un humain sur sa peau (cutis), comprenant, logés dans un boîtier rigide (200 a),
(a) au moins un moyen (201) pour émettre un rayonnement ayant une composition spectral définie sur la peau (cutis) ;
(b) au moins un capteur (202) pour mesurer la partie du rayonnement irradié par le moyen d'émission de rayonnement (201) qui est réfléchie par la peau (cutis) ; et
(c) au moins des moyens (212) pour fournir de l'énergie à l'au moins un moyen d'émission (201) et/ou à l'au moins un capteur (202) et pour transmettre des données mesurées à un moyen d'analyse de données (203) ;
ledit dispositif comprenant en outre
(d) dans ledit boîtier (200 a) : au moins un moyen (205) pour diffuser le rayonnement, émis par le moyen d'émission de rayonnement (201), entre le moyen d'émission de rayonnement (201) et la peau de l'humain (cutis) ;
(e) dans ledit boîtier (200 a) : au moins un moyen (202 a) pour focaliser, en direction de l'au moins un capteur (202), la partie du rayonnement irradié par le moyen d'émission de rayonnement (201) qui est réfléchie par la peau (cutis) ;
(f) dans ledit boîtier (200 a) : au moins un moyen (206) apte à fixer la position dudit au moins un moyen d'émission de rayonnement (201), dudit au moins un moyen de focalisation (202 a), dudit au moins un capteur (202), et dudit au moins un moyen de diffusion de rayonnement (205) les uns par rapport aux autres ; et
(g) au moins une surface plate et rigide (207) apte à permettre le passage du rayonnement émis à partir dudit au moins un moyen d'émission de rayonnement (201) en dehors dudit boîtier (200 a) en direction de la peau de l'humain (cutis) et du rayonnement réfléchi à partir de la peau de l'humain (cutis) dans ledit boîtier (200 a) en direction de l'au moins un moyen de focalisation (202 a) et de l'au moins un capteur (202), **caractérisé en ce que** ladite au moins une surface plate et rigide (207) est incluse dans l'agencement des quatre composants de (f) ci-dessus fixés par ledit moyen de fixation (206) ; ledit dispositif comprenant en outre
(h) une membrane flexible (208) qui incorpore ladite surface plate et rigide (207), et
(i) au moins un moyen (209) disposé à l'intérieur du boîtier (200 a) et apte à activer la mesure de l'état de la peau et/ou de la couleur de la peau de l'humain ;
ou
dispositif (200) pour une mesure de l'état et/ou de la couleur, pour autant que cela reste pertinent pour déterminer une dose de rayonnement actinique applicable, de la peau d'un humain sur sa peau (cutis), ledit dispositif (200) étant constitué par, un boîtier rigide (200 a), et
(a) logés dans le boîtier rigide (200 a): un ou plusieurs moyen(s) (201) pour émettre un rayonnement ayant une composition spectrale définie sur la peau (cutis) ;
(b) logés dans le boîtier rigide (200 a): un ou plusieurs capteur(s) (202) pour mesurer la partie du rayonnement irradié par le moyen d'émission de rayonnement (201) qui est réfléchie par la peau (cutis) ;
(c) logés dans le boîtier rigide (200 a): des moyens (212) pour fournir de l'énergie à l'un ou aux plusieurs moyen(s) d'émission (201) et/ou à l'un ou aux plusieurs capteur(s) (202) et pour transmettre des données mesurées à un moyen d'analyse de données (203) ;
(d) dans ledit boîtier (200 a) : un ou plusieurs moyen(s) (205) pour diffuser le rayonnement, émis par le moyen d'émission de rayonnement (201), entre le moyen d'émission de rayonnement (201) et la peau de l'humain (cutis) ;
(e) dans ledit boîtier (200 a) : un ou plusieurs moyen(s) (202 a) pour focaliser, en direction de l'un ou des plusieurs capteur(s) (202), la partie du rayonnement irradié par le moyen d'émission de rayonnement (201) qui est réfléchie par la peau (cutis) ;
(f) dans ledit boîtier (200 a) : un ou plusieurs moyen(s) (206) apte à fixer la position dudit un ou desdits plusieurs moyen(s) d'émission de rayonnement (201), dudit un ou desdits plusieurs moyen(s) de focalisation (202 a), dudit un ou desdits plusieurs capteur(s) (202), et dudit un ou desdits plusieurs moyen de diffusion de rayonnement (205) les uns par rapport aux autres ;
(g) une ou plusieurs surface(s) plate et rigide (207) apte à permettre le passage du rayonnement émis à partir dudit un ou desdits plusieurs moyen(s) d'émission de rayonnement (201) en dehors dudit boîtier (200 a) en direction de la peau de l'humain (cutis) et du rayonnement réfléchi à partir de la peau de l'humain (cutis) dans ledit boîtier (200 a) en direction de l'un ou des plusieurs moyen(s) de focalisation (202 a) et de l'un ou des plusieurs capteur(s) (202), et moyens (h) et (i), **caractérisé en ce que** ladite une ou lesdits plusieurs surface(s) plate et rigide (207) est/sont incluse(s) dans l'agencement des quatre composants de (f) ci-dessus fixés par ledit moyen de fixation (206) ; et par que
(h) une membrane flexible (208) incorpore ladite surface plate et rigide (207), et
(i) un ou plusieurs moyen(s) (209) est/sont disposé à l'intérieur du boîtier (200 a) et est/sont apte à activer la mesure de l'état de la peau et/ou de la couleur de la peau de l'humain.

2. Dispositif (200) selon la revendication 1, dans lequel ledit dispositif (200) comprend ladite membrane flexible (208) dans une connexion amovible audit boîtier (200 a).

3. Dispositif (200) selon la revendication 2, dans lequel ladite connexion amovible de la membrane flexible (208) au boîtier (200 a) est fermée hermétiquement pour être imperméable aux gaz, vapeurs, liquides et solides.

4. Dispositif (200) selon l'une quelconque des revendications 1 à 3, dans lequel ledit au moins un moyen d'activation (209) est disposé et fixé sur un côté du moyen de diffusion de rayonnement (205) qui est à l'opposé du côté de la surface plate et rigide (207), de préférence dans lequel ledit au moins un moyen d'activation (209) est disposé sur un côté du moyen de diffusion de rayonnement (205) qui est à l'opposé de la surface plate et rigide (207) et est disposé et fixé au centre sur l'axe longitudinal central (A) du boîtier (200 a).

5. Dispositif (200) selon la revendication 4, en cas que le dispositif comprend autre éléments, ledit dispositif comprenant en outre au moins un aimant (210) apte à effectuer un contact pour activer ledit moyen d'activation (209), de préférence comprenant un ou des aimants (210) en association avec un capteur à effet Hall en tant que moyen d'activation (209).

6. Dispositif (200) selon l'une quelconque des revendications 1 à 5, en cas que le dispositif comprend autre éléments, ledit dispositif comprenant en outre le boîtier rigide ayant la forme d'un cône tronqué, de préférence ayant des zones de saisie en couleur (230), plus préférablement ayant des zones de saisie (230) en un matériau transparent à la lumière, encore plus préférablement ayant des zones de saisie (230) en un matériau apte à permettre à la lumière d'éclairage en couleur de passer de l'intérieur au côté extérieur, le plus préférablement ayant des zones de saisie (230) en un matériau apte à permettre à la lumière d'éclairage en couleur de passer de l'intérieur, lorsque des signaux électroniques relatifs à des fonctions de mesure leur sont adressés, au côté extérieur, signalant ainsi des fonctions de mesures à l'utilisateur.

7. Appareil (100) pour irradier un rayonnement actinique sur le corps d'un utilisateur, ledit appareil comprenant au moins un moyen (106) ayant une surface (102) qui fait face à l'utilisateur et est transparente au rayonnement actinique et ayant plusieurs groupes de sources de rayonnement actinique (104) agencés sur le côté de la surface (102) qui est opposé à l'utilisateur, lesquelles sources de rayonnement actinique (104) sont conçues, au regard de leur nombre et de leur position, de telle sorte qu'elles peuvent diriger un rayonnement actinique sensiblement séparément sur différentes parties du corps de l'utilisateur, et peuvent être réglées par un ou plusieurs moyens (108), pour régler la puissance des sources de rayonnement actinique (104) dans lequel le réglage de la puissance est effectué conformément aux résultats des mesures de l'état, pour déterminer une dose de rayonnement actinique applicable, de la peau de l'utilisateur au niveau de plusieurs points de son cutis, au moyen d'au moins un dispositif selon l'une quelconque ou plusieurs des revendications 1 à 6.

8. Appareil (100) selon la revendication 7, dans lequel le rayonnement actinique est un rayonnement actinique comprenant au moins partiellement un rayonnement dans la plage de longueurs d'onde UV ayant un effet physiologique, de préférence un effet bronzant, sur la peau de l'utilisateur (cutis) ; et/ou dans lequel le rayonnement actinique est un rayonnement actinique comprenant au moins partiellement un rayonnement dans la plage des longueurs d'onde visibles ayant un effet physiologique sur la peau de l'utilisateur (cutis) ; et/ou dans lequel le rayonnement actinique est un rayonnement actinique comprenant au moins partiellement un rayonnement dans la plage des longueurs d'onde IR ayant un effet physiologique sur la peau de l'utilisateur (cutis) ; et/ou dans lequel le rayonnement actinique est un rayonnement actinique comprenant au moins partiellement un rayonnement dans au moins deux plages de longueurs d'onde sélectionnées parmi les longueurs d'onde UV ayant un effet physiologique sur la peau de l'utilisateur (cutis), la plage des longueurs d'onde visibles ayant un effet physiologique sur la peau de l'utilisateur (cutis), et la plage des longueurs d'onde IR ayant un effet physiologique sur la peau de l'utilisateur (cutis).

9. Appareil (100) selon l'une quelconque ou plusieurs des revendications 7 ou 8, dans lequel l'au moins un moyen (200) est/sont apte(s) à réaliser une mesure de la couleur de la peau de l'utilisateur et/ou de l'état de la peau de l'utilisateur avant de commencer l'irradiation.

10. Appareil (100) selon la revendication 9, dans lequel l'au moins un moyen (200) est/sont apte(s) à réaliser des mesures supplémentaires de la couleur de la peau de l'utilisateur et/ou de l'état de bronzage de la peau de l'utilisateur - éventuellement en plus - au moins une fois au cours de l'irradiation, de préférence de manière intermittente ou continue au cours de l'irradiation.

11. Appareil (100) selon l'une quelconque ou plusieurs des revendications 7 à 10, comprenant plusieurs moyens (108) pour régler la puissance des sources de rayonnement UV (104) conformément aux, et sous l'influence directe des, résultats des mesures du type de la peau de l'utilisateur et/ou de l'état de la peau de l'utilisateur au niveau de plusieurs points de la peau de l'utilisateur (cutis), de préférence sélectionnés parmi la peau du visage de l'utilisateur, la peau du thorax de l'utilisateur, la peau du dos de l'utilisateur, la peau du ventre de l'utilisateur, la peau du fessier de l'utilisateur, la peau des bras de l'utilisateur et la peau des jambes de l'utilisateur.

12. Appareil (100) selon l'une quelconque ou plusieurs des revendications 7 à 11, dans lequel la mesure de la couleur de la peau de l'utilisateur et/ou de l'état de la peau de l'utilisateur est effectuée au moyen d'un ou de plusieurs moyens (200) selon l'une quelconque ou plusieurs des revendications 1 à 6, et les moyens (108) réglant la puissance des sources de rayonnement actinique (104) sont commandés directement conformément aux résultats de ladite mesure de la couleur de la peau de l'utilisateur etlou de l'état de la peau de l'utilisateur.

13. Appareil (100) selon l'une quelconque des revendications 7 à 12, comprenant
(a) un lit (110) avec une surface (112) pour l'utilisateur transparente au rayonnement actinique, et des sources de rayonnement actinique (114) émettant un rayonnement actinique ;
(b) une partie supérieure mobile (120) ayant une surface de protection (122) transparente au rayon actinique, et des sources de rayonnement actinique (124) émettant un rayonnement actinique ;
(c) dans lequel la partie supérieure (120), en liaison avec le lit (110), peut être ouverte pour permettre à un utilisateur d'y entrer et peut être fermée avant de commencer le processus d'irradiation ;
(d) des moyens de réglage (130) pour régler la durée d'un processus d'irradiation qui peuvent être utilisés par l'utilisateur ; et
(e) un ou plusieurs dispositifs (108) pour régler séparément la puissance des sources de rayonnement actinique (114, 124), où le réglage de la puissance est effectué conformément aux résultats des mesures de la couleur de la peau de l'utilisateur et/ou de l'état de la peau de l'utilisateur au niveau de plusieurs points de sa peau (cutis) au moyen du dispositif 200 selon l'une quelconque des revendications 1 à 6.

14. Dispositif (200) selon l'une quelconque des revendications 1 à 7 destiné à être utilisé dans un processus d'irradiation, sur le corps d'un utilisateur, d'un rayonnement actinique provenant de sources de rayonnement actinique émettant un tel rayonnement, ledit dispositif (200) étant apte à permettre les étapes de mesure de l'état et/ou de la couleur, pour déterminer une dose d'irradiation actinique applicable, de la peau de l'utilisateur au niveau de plusieurs points de la peau (cutis), et de réglage de la puissance de groupes séparés desdites sources de rayonnement actinique aptes à diriger leur rayonnement sensiblement séparément sur différentes parties du corps de l'utilisateur, conformément aux résultats desdites mesures effectuées au moyen dudit dispositif (200).

15. Dispositif (200) selon la revendication 14 destiné à être utilisé dans un processus d'irradiation, sur le corps d'un utilisateur, d'un rayonnement actinique provenant de sources de rayonnement actinique émettant un tel rayonnement, ledit dispositif (200) étant apte à permettre les étapes de mesure de la couleur de la peau ou de l'état de la peau de l'utilisateur au niveau d'au moins deux points de la peau (cutis) et de réglage de la puissance de deux ou de plus de deux groupes de sources de rayonnement actinique conformément aux résultats desdites étapes de mesure, de préférence étant apte à permettre les étapes de mesure de la couleur de la peau ou de l'état de la peau de l'utilisateur au niveau d'au moins deux points de la peau (cutis), qui sont sélectionnés parmi la peau du visage, la peau du thorax, la peau du dos, la peau du ventre, la peau du fessier, la peau des bras et la peau des jambes.

16. Dispositif (200) selon la revendication 14 ou la revendication 15 destiné à être utilisé dans un processus d'irradiation, sur la peau d'un utilisateur, d'un rayonnement actinique provenant de sources de rayonnement actinique émettant un tel rayonnement, ledit dispositif (200) étant apte à permettre l'étape de mesure de la couleur/du type de la peau de l'utilisateur et/ou de l'état de la peau de l'utilisateur avant de commencer l'irradiation, ou ledit dispositif (200) étant apte à permettre l'étape de mesure de la couleur/du type de la peau de l'utilisateur et/ou de l'état de la peau de l'utilisateur en plus au moins une fois au cours de l'irradiation, de préférence de manière intermittente ou continue au cours de l'irradiation, avec un rayon actinique.

17. Dispositif (200) selon l'une quelconque des revendications 14 à 16 destiné à être utilisé dans un processus d'irradiation de la peau à des fins cosmétiques ou destiné à être utilisé dans un processus d'irradiation de la peau à des fins médicales.
